# EUROPEAN PATENT APPLICATION

(11) **EP 3 266 785 A1**
(43) Date of publication of application: **10.01.2018**
(21) Application number: 15875212.1
(22) Date of filing: 29.12.2015
(51) Int. Cl.: C07D 487/04, A61K 31/519, A61P 31/00, A61P 37/00, A61P 35/00, A61P 11/00

(54) **TOLL-LIKE RECEPTOR-7 AGONIST**

(30) Priority: 29.12.2014 CN 201410837925
(71) Applicant: Medshine Discovery Inc., Nanjing, Jiangsu 210032 (CN)
(72) Inventor: DING, Charles Z., Shanghai 200131 (CN); SUN, Fei, Shanghai 200131 (CN); WU, Lifang, Shanghai 200131 (CN); DU, Jinhua, Shanghai 200131 (CN); KATSU, Yasuhiro, Shanghai 200131 (CN); HU, Guoping, Shanghai 200131 (CN); LI, Jian, Shanghai 200131 (CN)
(74) Representative: Haseltine Lake LLP
(86) International application number: PCT/CN2015/099375
(87) International publication number: WO 2016/107536

(57) **Abstract**

Disclosed are a novel pyrrolopyrimidine ring compound as a TLR7 agonist or a pharmaceutically acceptable salt thereof, used for preventing or treating allergic rhinitis and asthma. In particular, disclosed is a compound represented by formula (I) or a pharmaceutically acceptable salt thereof

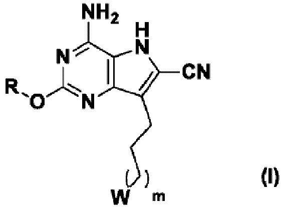

## Description

### Field of invention

The present invention relates to a novel pyrrolopyrimidine compound as an agonist of TLR7, pharmaceutically acceptable salt, hydrate or prodrug thereof, which is useful to prevent or treat allergic rhinitis and asthma. Especially, it relates to a compound of formula (I), pharmaceutically acceptable salt, hydrate or prodrug thereof.

### Prior arts

Toll-like receptors (TLRs) play an important role in natural immunity against microbial infection. The function of TLRs predominantly expressed by activating innate immune cells where their role is to monitor the environment for signs of infection and mobilising defence mechanisms aimed at the elimination of invading pathogens. This results in a variety of cellular responses including the production of interferon (IFNs), pro-inflammatory cytokines and effector cytokines that direct the adaptive immune response.

Administration of a small molecule compound which could stimulate the innate immune response, including the activation of type I interferon and other cytokines via Toll-like receptors could become an important strategy for the treatment or prevention of human diseases. Small molecule agonists of TLR7 have been described which can induce interferon alpha in animals and in man *(*Takeda K. eta I, Annu. Rev. Immunol; 2003:21, 335-76*).* The profile of the response seen from different TLR agonists depends on the cell type activated.

TLR7 and TLR8 are highly homologous. Stimulation of TLR7 can induce the generation of interferon alpha in man and animal which can treat allergic diseases and other inflammatory disease, such as allergic asthma and rhinitis, whereas stimulation of TLR8 mainly leads to the generation of pro-inflammatory cytokine, such as tumor necrosis factor-α (TNF-α) and chemokines etc, which can lead to serious side-effects. Therefore, to improve the selectivity between TLR7 and TLR8 is critical for the safety of TLR7 agonists.

Allergic diseases are mainly caused by cytokine secretion disorders which are related to antigen specific Th2 cells, and Th2 immune responses to the allergen are associated with raised levels of IgE, which, *via* its effects on mast cells, promotes a hypersensitivity to allergens, resulting in the symptoms seen. In healthy individuals the immune-response (Th2/Th1) to allergens is more balanced, TLR7 ligands have been shown to reduce Th2 cytokine and enhance Th1 cytokine release and to ameliorate Th2-type inflammatory responses in allergic lung models *in vivo (*Fi\i L, et al, J. All. Clin. Immunol., 2006: 118, 511-517; Moisan J., et al, Am. J. Physiol. Lung Cell Mol. Physiol., 2006: 290, L987-995; Tao et a\, Chin. Med. J., 2006: 119, 640-648). Thus, TLR7 ligands have the potential to rebalance the immune-response seen in inflammatory individuals and lead to disease modification.

Recent clinical studies on TLR7 agonist have shown repeated intranasal stimulation produces a sustained reduction in the responsiveness to allergen in patients with both allergic rhinitis and allergic asthma *(*GreiffL. Respiratory Research, 2012:13, 53*;* Leaker B.R. eta I, Am. J. Respir. Crit Care Med., 2012:185, A4184*).* Several TLR7 agonists have been reported, such as imiquimod, resiquimod. But novel TLR7 agonists with preferred selectivity, potency and safety profile are still highly desired.

A series of TLR7 agonists were referred to WO2014/081645, where it describes treating infectious diseases, such as allergic rhinitis and asthma, by compounds of formula (II) 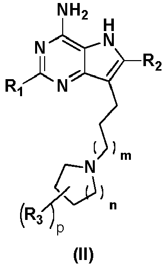 wherein R₁ is an unsubstituted C₄₋₆ alkyl or a C₁₋₂ alkoxy C₁₋₂ alkyl-; R₂ is a hydrogen or a methyl; R₃ is a hydrogen, a halo or a C₁₋₃ alkyl; m is an integer having a value of 2 to 4; n is an integer having a value of 0 to 3; p is an integer having a value of 0 to 2.

GSK-2245035 is shown as formula (III):

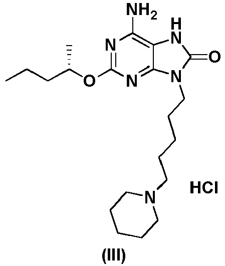

R848 is shown as formula (IV)

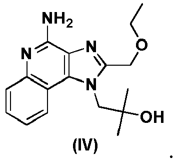

### Content of the present invention

The present invention provides a compound of formula (I) or a pharmaceutically acceptable salt, a hydrate or a prodrug thereof, 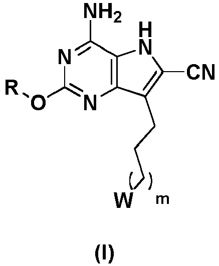 wherein,
R is a C₃₋₈ alkyl which is optionally substituted;
m is 0, 1, 2, 3, or 4;
W is an optionally substituted C₃₋₈ heteroalkyl, an optionally substituted 4∼12 membered cycloalkyl, an optionally substituted 4∼12 membered heterocycloalkyl, or an optionally substituted amino acids;
the "hetero" represents heteroatom or heteroatomic group, which is independently selected from the group consisting of O, S, N, C(=O), S(=O), and S(=O)₂;
the number of the heteroatom or the heteroatomic group is independently 0, 1, 2, 3 or 4.

In one embodiment of the present invention, the substituent of C₃₋₈ alkyl, C₃₋₈ heteroalkyl, 4-12 membered cycloalkyl, or 4-12 membered heterocycloalkyl is independently selected from the group consisting of F, Cl, Br, I, OH, CN, NH₂, NH₂(C=O), C₁₋₄ alkyl, or C₁₋₄ heteroalkyl, 4-6 membered alkyl or heteroalkyl, 4-6 membered heteroalkyl-C(=O)-, the 4-6 membered alkyl or heteroalkyl is optionally substituted by halogen, NH₂, OH, CN, or C₁₋₄ alkyl, the "hetero" is defined as above.

In one embodiment of the present invention, the substituent of C₃₋₈ alkyl, C₃₋₈ heteroalkyl, 4-12 membered cycloalkyl, or 4-12 membered heterocycloalkyl is independently selected from the group consisting of F, Cl, Br, I, OH, CN, NH₂, NH₂(C=O), Me, Et, 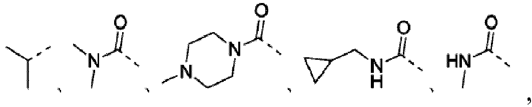 the "hetero" is defined as above.

In some embodiments of the present invention, the number of the substituent is independently selected from 0, 1, 2, or 3.

In some embodiments of the present invention, the C₃₋₈ alkyl is selected from the group consisting of

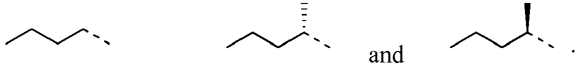

In some embodiments of the present invention, the C₃₋₈ heteroalkyl is -N(C₁₋₄ alkyl)(C₁₋₄ alkyl).

In some embodiments of the present invention, the 4-12 membered cycloalkyl or 4-12 membered heterocycloalkyl is 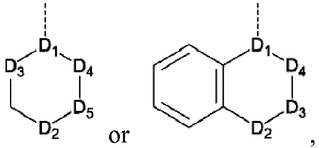 D₁ is N or C(Rₐ), D₂₋₅ is independently selected from the group consisting of O, S, [C(Rₐ)(R_{b})]₁₋₂ and N(R_{c}), one or both of D₃ and D₄ may be single bond(s), Rₐ, R_{b} or R_{c} is independently selected from the group consisting of H, C₁₋₄ alkyl, C₁₋₄ heteroalkyl, halogen, OH, CN, NH₂(C=O), any Rₐ and R_{b} may be optionally attached to one atom to form a 4-6 membered cycloalkyl, 4-6 membered oxa-cycloalkyl, 4-6 membered aza-cycloalkyl, the 4-6 membered cycloalkyl is optionally substituted by 1 to 3 of C₁₋₄ alkyl.

In some embodiments of the present invention, W is selected from the group consisting of

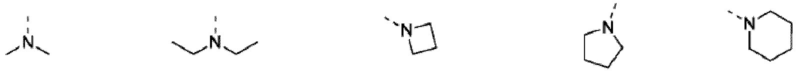

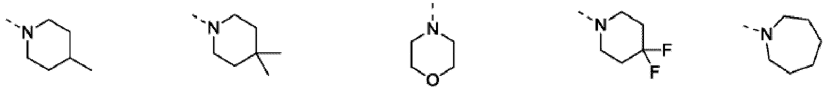

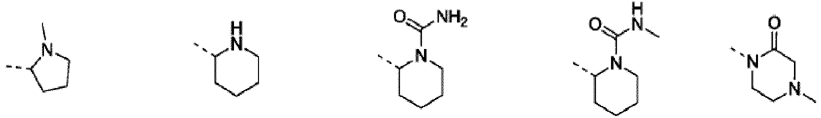

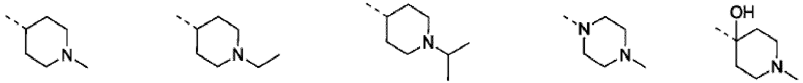

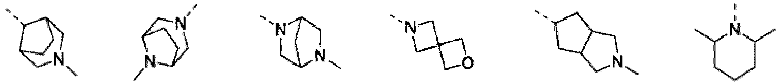

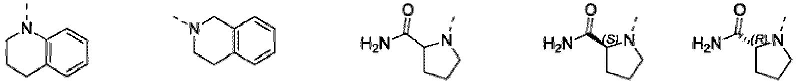

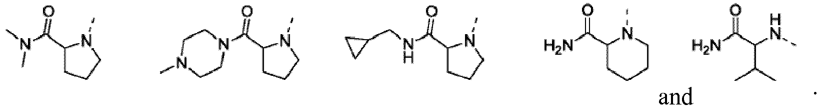

In some embodiments of the present invention, the compound is selected from the group consisting of

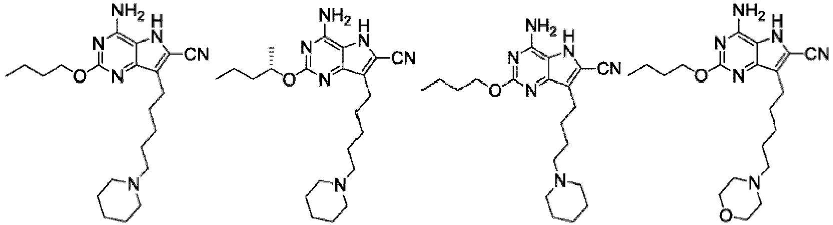

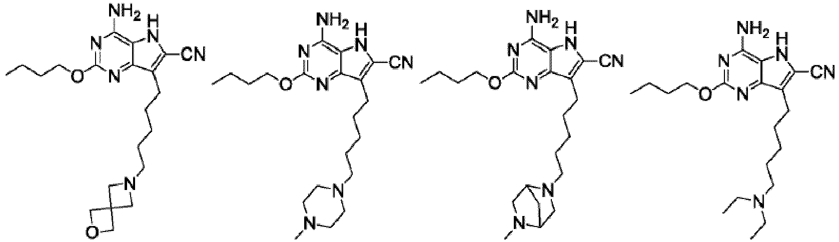

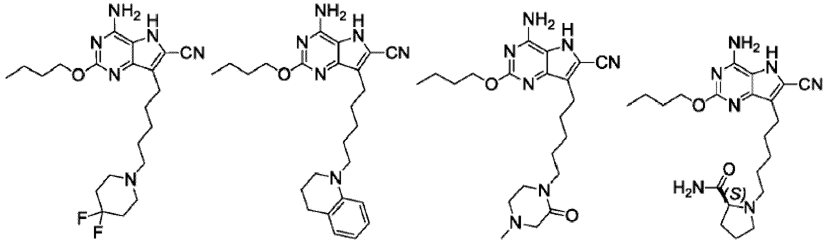

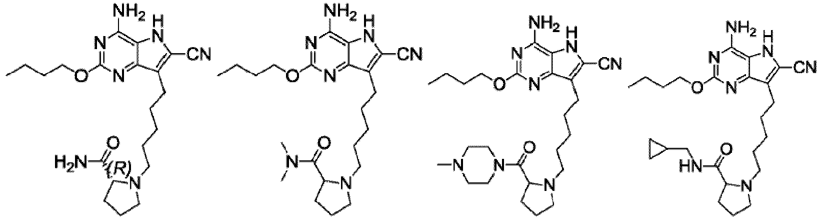

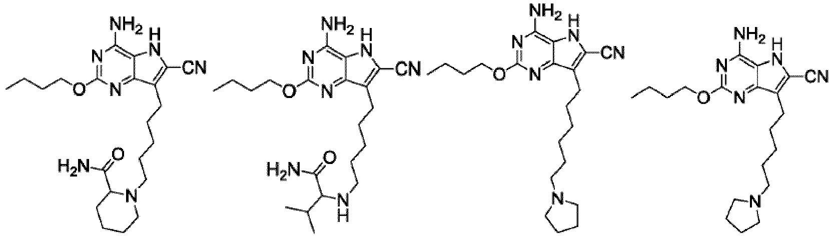

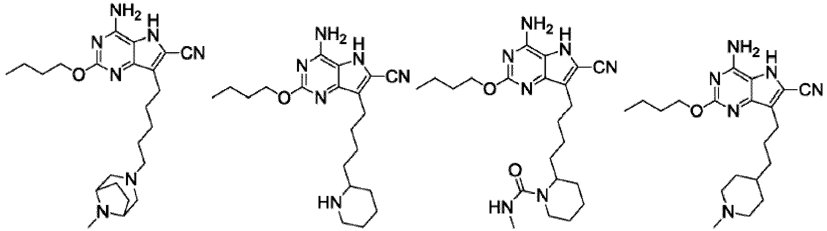

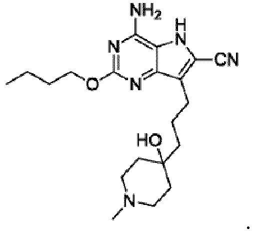

The present invention also provides two processes for preparing the compound of formula (I), which are shown as below respectively,
process 1: 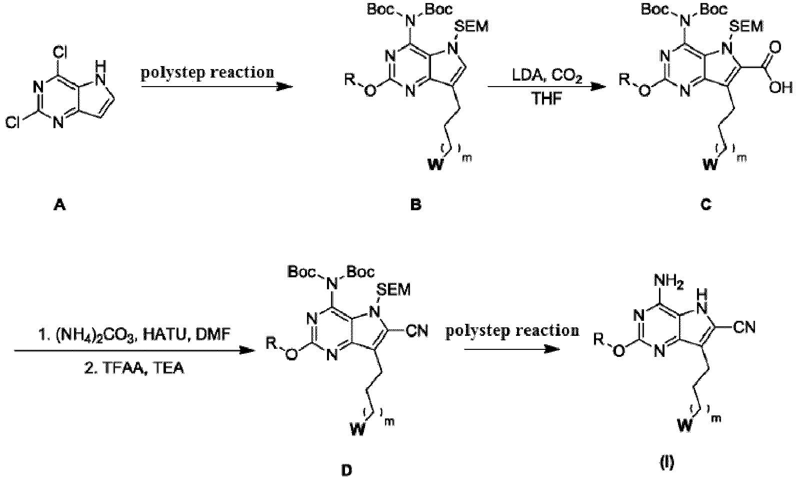 process 2: 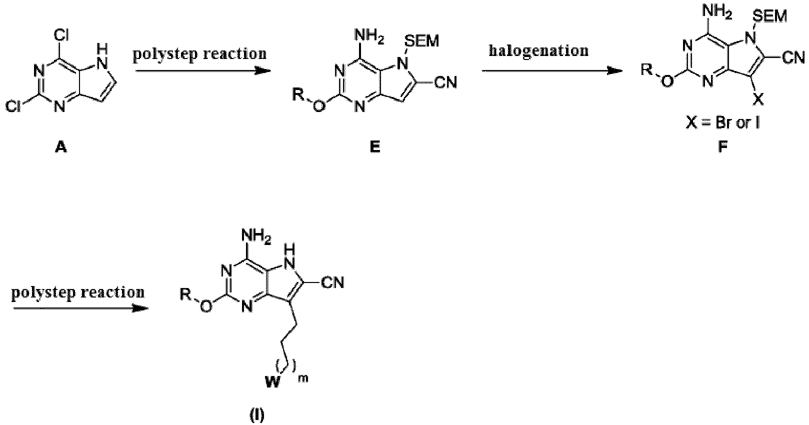 wherein, SEM represents 2-(trimethylsilyl)ethoxymethyl, which is an amino protecting group.

The present invention also provides a use of the compound, pharmaceutically acceptable salt, hydrate, or prodrug thereof in manufacturing a medicament for the prevention or treatment of allergic diseases and other inflammatory conditions, infection diseases or cancers.

In some embodiments of the present invention, the disease is allergic rhinitis.

In some embodiments of the present invention, the disease is asthma.

### Definitions

Unless otherwise specified, the following terms and phrases used herein are intended to have the following meanings. It is understood that a specific term or phrase which is not specifically defined should not be considered indefinite or unclear, but should be construed according to their conventional meaning. Commercial names used herein refer to the corresponding product or the active ingredients thereof.

The term "treating" or grammatical equivalents thereof, when used in "treating a disease", means slowing or stopping the development of a disease, or ameliorating at least one symptom of a disease, more preferably ameliorating more than one symptoms of a disease.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problems or complications, commensurate with a reasonable benefit/risk ratio.

The term "pharmaceutically acceptable salt" is meant to include a salt of the compound of the present invention, which is prepared by a relatively nontoxic acid or base and the compound of the present invention having specific substituents. When the compound of the present invention contains a relatively acidic functional group, a base addition salt can be obtained by contacting a neutral form of such compounds with a sufficient amount of a desired base, either neat or in a suitable inert solvent. Examples of the pharmaceutically acceptable base addition salts include salts of sodium, potassium, calcium, ammonium, organic amine, or magnesium, or the similar. When the compound of the present invention contains a relatively basic functional group, an acid addition salt can be obtained by contacting a neutral form of such compounds with a sufficient amount of a desired acid, either neat or in a suitable inert solvent. Examples of the pharmaceutically acceptable acid addition salts include salts of inorganic acids, the inorganic acids include hydrochloric, hydrobromic, nitric, carbonic, hydrocarbonic, phosphoric, hydrophosphoric, dihydrophosphoric, sulfuric, hydrosulfuric, hydriodic, or phosphorous acid and the like; as well as salts of organic acids, the organic acids include formic, acetic, propionic, isobutyric, maleic, malonic, benzoic, succinic, suberic, fumaric, lactic, mandelic, phthalic, benzenesulfonic, *p*-tolylsulfonic, citric, tartaric, methanesulfonic acid, or the like; and also salts of amino acids (such as arginate and the like), and salts of organic acids like glucuronic acid and the like (see Berge et al., "Pharmaceutical Salts", Journal of Pharmaceutical Science 66: 1-19 (1977)). Certain specific compounds of the present invention contain both basic and acidic functionalities that allow the compounds to be converted into either base or acid addition salts.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture thereof. Generally, non-aqueous media like ether, ethyl acetate, ethanol, isopropanol, or acetonitrile or the like is preferred.

In addition to salt forms, the present invention provides compounds which are in a prodrug form. Prodrugs of the compounds described herein readily undergo chemical changes under physiological conditions to provide the compounds of the invention. Additionally, prodrugs can be converted to the compounds of the invention by chemical or biochemical methods *in vivo.*

Certain compounds of the present invention can exist in non-solvated forms as well as solvated forms, including hydrated forms. In general, the solvated forms are equivalent to non-solvated forms and are encompassed within the scope of the present invention. Certain compounds of the present invention may be present in crystalline or amorphous form.

The term "pharmaceutically acceptable carrier" refers to any formulation or carrier medium that is capable of delivery of an effective amount of an active agent of the present invention without toxic side effects in a host or patient and without interfering the bioactivity of the active agent. Representative carriers include water, oils, both vegetable and mineral, cream bases, lotion bases, ointment bases and the like. These bases include suspending agents, thickeners, penetration enhancers, and the like. The term "excipients" is conventionally known to mean carriers, diluents and/or vehicles used in formulating drug compositions effective for the desired use.

For drugs or pharmacologically active agents, the terms "effective amount" or "therapeutically effective amount" refers to a nontoxic but sufficient amount of the drug or formulation to provide the desired effect. An "effective amount" of an active agent of the composition refers to the amount of the active agent required to provide the desired effect when used in combination with the other active agent of the composition. The amount that is "effective" will vary from subject to subject, depending on the age and general condition of a recipient, and also a particular active agent, and an appropriate effective amount in an individual case may be determined by one of ordinary skill in the art using routine experimentation.

The terms "active ingredient," "therapeutic agent," "active substance," or "active agent" mean a chemical entity which can be effective in treating disorder, disease or condition in a subject.

The term "substituted" means that any one or more than one hydrogen atoms attached to one specific atom is replaced with substituent(s). The term "optionally substituted" means that the designated atom can be substituted or unsubstituted, and unless otherwise stated, the species and number of the substituents may be arbitrary provided that they can be achieved in chemistry.

The term "alkyl" refers to a saturated, hydrocarbon chain having a specified number of atoms. Unless otherwise stated, the term 'alkyl' includes linear and branched alkyl groups. For example, C₁₋₆ alkyl refers to a saturated, linear or branched hydrocarbon chain having 1, 2, 3, 4, 5 or 6 carbon atoms, such as ethyl and isopropyl, and *n*-C₃₋₆ alkyl refers to a saturated, linear hydrocarbon chain having from 3 to 6 carbon atoms, such as *n*-propyl and *n*-butyl.

Unless otherwise stated, the term "hetero" means heteroatom or heteroradical (i.e. a radical containing heteroatom), including atoms other than carbon (C) and hydrogen (H), also including the radicals containing these heteroatoms. Examples include oxygen (O), nitrogen (N), sulfur (S), boron (B), -O-, -S-, =O, =S, -C(=O)O-, -C(=O)-, -C(=S)-, -S(=O), -S(=O)₂-, and optionally substituted -C(=O)N(H)-, optionally substituted -N(H)-, optionally substituted -C(=NH)-, optionally substituted -S(=O)₂N(H)-, or optionally substituted -S(=O)N(H)-.

Unless otherwise stated, the term "heteroalkyl", when used alone or in combination with other terms, means a stable linear or branched chain, or cyclic hydrocarbon radical, or combinations thereof, consisting of the stated number of carbon atoms and at least one heteroatom. In an exemplary embodiment, the heteroatoms can be selected from the group consisting of O, N and S, and wherein the nitrogen and sulfur atoms may optionally be oxidized and the nitrogen atom may optionally be quaternized. The O, N and S atoms may be placed at any interior position of the heteroalkyl or placed between the alkyl and the remainder of the molecule.

Unless otherwise specified, "ring" as used herein means a substituted or unsubstituted cycloalkyl, heterocycloalkyl, cycloalkenyl, heterocycloalkenyl, cycloalkynyl, heterocycloalkynyl, aryl, or heteroaryl. The ring includes mono, bi, sprio, fused, or bridged ring moieties. The number of atoms in a ring is typically defined by the number of members in the ring. For example, a "5- to 11-membered ring" means there are 5, 6, 8, 9, 10 or 11 atoms in the encircling arrangement. Unless otherwise specified, the ring optionally includes 1, 2, or 3 heteroatoms. Thus, the term "5- to 11-membered ring" includes, for example phenyl, pyridyl and piperidyl. The term "5- to 11-membered heterocycloalkyl ring", on the other hand, includes pyridyl and piperidyl, but excludes phenyl. The term "ring" further includes a ring system comprising at least one "ring", wherein each "ring" is independently defined as above.

The term "halo" or "halogen" means fluorine, chlorine, bromine, and iodine atom.

The term "protecting group" includes but not limited to "amino-protecting group". The term "amino-protecting group" means a protecting group suitable for preventing undesired reactions at an amino nitrogen. Representative amino-protecting groups include, but not limited to, formyl; acyl, for example alkanoyl, such as acetyl etc.; alkoxycarbonyl, such as *tert*-butoxycarbonyl(Boc) etc.; arylmethoxycarbonyl, such as benzyloxycarbonyl(Cbz) and 9-fluorenylmethoxycarbonyl(Fmoc) etc.; arylmethyl, such as benzyl(Bn) etc.; silicyl, such as trimethylsilyl (TMS) and *tert*-butyldimethylsilyl (TBS) and the like.

Certain compounds in the present invention may include chiral atoms and multiple bonds, and thus one or more stereoisomeric forms may occur. Including stereoisomers of compounds in previous studies, both as individual and mixtures, where racemic is also included.

Certain compounds of the present invention may be in the form of all tautomers, including all tautomers of the compound in previous study whether the compound is as an individual or as a mixture.

The term "polymorph" refers to the property that the compound of the present invention may have one or more than one crystal structure. The compounds of the present invention may exist as various crystalline or amorphous forms, or as solid plugs, powders, films by employing processes such as precipitation, crystallization, freeze drying, spray drying, or evaporative drying and so on. They may be administered alone or in combination with one or more than one other prior compounds, or in combination with one or more than one other drugs. Generally, they will be administered as a formulation in association with one or more than one acceptable excipients.

The compounds of the present invention can be prepared in a number of ways known to one skilled in the art, including the processes described below, the embodiment formed together with synthetic methods known in the art, and variations thereon as appreciated by those skilled in the art. Preferred embodiments include, but not limited to the embodiments of the present invention.

The reactions of the embodiments of the present invention are performed in a solvent appropriate to the reagents and materials employed and suitable for the transformations being effected. To achieve the compounds of the present invention, some modifications or screenings are required to the synthesis steps or reaction routes based on the present embodiments for the person skilled.

Solvents used can be commercially available.

Reactions typically run in anhydrous solvents under an inert atmosphere of nitrogen. Proton NMR is recorded on Bruker Avance III 400 (400 MHz) spectrometer and chemical shifts are reported as (ppm) in down field from tetramethylsilane. Mass spectra are determined on Agilent 1200 series plus 6110 (&1956A). LC/MS, or Shimadzu MS consisting of a DAD: SPD-M20A (LC) and Shimadzu Micromass 2020 detector. The mass spectrometer is equipped with an electrospray ion source (ESI) operated in a positive or negative mode.

The following abbreviations are used herein: aq. is aqueous; eq. is equivalent; SEM-Cl is (2-(chloromethoxy)ethyl)trimethylsilane; SEM is (2-(chloromethoxy)ethyl)trimethylsilyl, which is an amino-protecting group; *i*-PrOH is propan-2-ol; DCM is dichloromethane; PE is petroleum ether; DMF is N, N-dimethylformamide; EtOAc or EA is ethyl acetate; EtOH is ethanol; MeOH is methanol; THF is tetrahydrofuran; DMSO is dimethyl sulfoxide; MeCN or ACN is acetonitrile; dioxane is 1,4-dioxane; Boc₂O is di-*tert*-butyl dicarbonate; BOC is *tert*-butoxycarbonyl, which is an amino-protecting group; CuI is copper(I) iodide; Pd(OH)₂/C is palladium hydroxide/carbon; Pd/C is palladium/carbon; (NH₄)₂CO₃ is ammonium carbonate; NaH is sodium hydride; NH₃•H₂O is aqueous ammonia; Na is sodium metal; HATU is O-(7-Aza-1H-benzotriazol-1-yl)-N,N,N',N'-tetramethyluronium hexafluorophosphate; DMAP is N,N-dimethylpyridin-4-amine; TFA is trifluoroacetic acid; TFAA is trifluoroacetic acid anhydride; TEA is triethylamine; DIEA or DIPEA is N,N-diisopropylethylamine; CO₂ is gaseous or solid carbon dioxide; NIS is *N-*iodosuccinimide; Pd(PPh₃P)₂Cl₂ is bis(triphenylphosphino) palladium chloride; Pd(PPh₃)₄ is. tetrakis(triphenylphosphine)palladium; Pd(t-Bu₃P)₂ is bis(tri-*t*-butylphosphine)palladium(0); LDA is lithium diisopropylamide; HCHO is formaldehyde; NaBH(AcO)₃ is sodium triacetoxyborohydride; HOBt is 1-hydroxybenzotriazole; EDCI is N-(3-dimethylaminopropyl)-N'-ethylcarbodiimide hydrochloride; DMP is Dess-Martin oxygenant; m.w. or microwave is microwave reaction; r.t. is room temperature.

Compounds are named either manually or by using ChemDraw®, or using vendors catalogue name if commercially available.

High-performance liquid chromatography analysis was performed on Shimadzu LC20AB system equipped with Shimadzu SIL-20A autosampler and Shimadzu DAD: SPD-M20A detector, with Xtimate C18 column (3µm, size: 2.1 × 300mm). Method of 0-60AB_6 min: linear gradient from 100% A solvent (0.0675% TFA aqueous solution) to 60% B solvent (0.0625% TFA in MeCN solution) was applied for 4.2min, followed by additional 1-min elution with 60% B solvent. Column pressure was re-equilibrated for 0.8min and eluent ratio (A:B) was adjusted to 100:0, and overall run time was 6 min. Method of 10-80AB_6 min: linear gradient from 90% A solvent (0.0675% TFA aqueous solution) to 80% B solvent (0.0625% TFA in MeCN solution) was applied for 4.2min, followed by additional 1-min elution with 80% B solvent. Column pressure was re-equilibrated for 0.8 min and eluent ratio (A:B) was adjusted to 90:10, and total run time was 6 min. Column temperature was 50°C, flow rate was 0.8mL/min, and diode array detection wavelength range was 200-400nm.

Thin-layer chromatography (TLC) was performed on Sanpont-group GF254 silica gel plate. Spots were usually visualized by UV-irradiation, or other methods for visualizing compound spots on developed TLC plate can also be used. In these methods: I₂ (1g of I₂ thoroughly mixed in 10g of silica gel), *p*-anisaldehyde (1g of *p*-anisaldehyde dissolved in 100mL of 10% H₂SO₄), ninhydrin (supplied by Aldrich), or special reagent ((NH₄)₆Mo₇O₂₄₈•4H₂O and 5g of (NH₄)₂Ce(IV)(NO₃)₆ dissolved in 450mL of H₂O and 50mL of H₂SO₄ (conc.)) was used to visualize the compound points. Flash column chromatography was performed on Silicycle 40-63µm (230-400mesh) silica gel, by the similar methods in the publication: Still, W. C.; Kahn, M.; Mitra, M. J. Org. Chem. 1978, 43, 2923-2925. Eluent of flash column chromatography or TLC included mixed solvents of dichloromethane/methanol, ethyl acetate/methanol and ethyl acetate/n-hexane.

Preparative chromatography analysis was performed on Gilson-281 Prep LC 322 system with Gilson UV/VIS-156 detector. Column was Agella Venusil ASB Prep C18, 5µm, 150 × 21.2mm, Phenomenex Gemini C18, 5µm, 150 × 30mm, Boston Symmetrix C18, 5µm, 150 × 30mm, or Phenomenex Synergi C18, 4µm, 150 × 30mm. At a flow rate of 25mL/min, compounds were eluted with MeCN/H₂O with slowly increasing gradient. Eluents in details were as follows:
HCl method: MeCN/H₂O (0.05% (v/v) HCl (aq, w%: 33-37.5%))
Formic acid method: MeCN/H₂O (0.2255% (v/v) formic acid)
Trifluoroacetic acid method: MeCN/H₂O (0.075% (v/v) trifluoroacetic acid)
Basic aqueous ammonia method: MeCN/H₂O (0.05% (v/v) NH₃•H₂O (aq, w%: 25-28%))
Total time was 8-15 min.

Compared to WO2014/081645, distinguishing features of the compounds in the present invention include (1) R₁ in WO2014/081645 is an alkyl group, but corresponding to an alkoxy group in compounds of the present invention. The present invention demonstrates that replacement of alkyl with alkoxy group can prominently improve drug efficacy and pharmacokinetics. (2) R₂ in WO2014/081645 is hydrogen or methyl, but corresponding to nitrile group in compounds of the present invention. The present invention demonstrates that nitrile group is crucial in drug efficacy improvement. The synthetic processes of both are significantly different, and the challenge became more.

After validation, compounds of the present invention are highly effective in inducing IFN-α generation, and can be utilized to prevent or treat allergic rhinitis and asthma, virus infection, or cancer and so on.

### Detailed description of the preferred embodiment

In order to illustrate the present invention in details, the invention will hereinafter be illustrated with reference to the following, non-limiting examples.

Formula (I) was synthesized according to the following general routes.

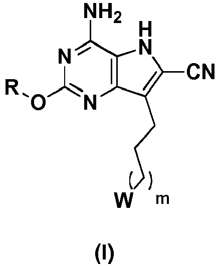

### General route 1:

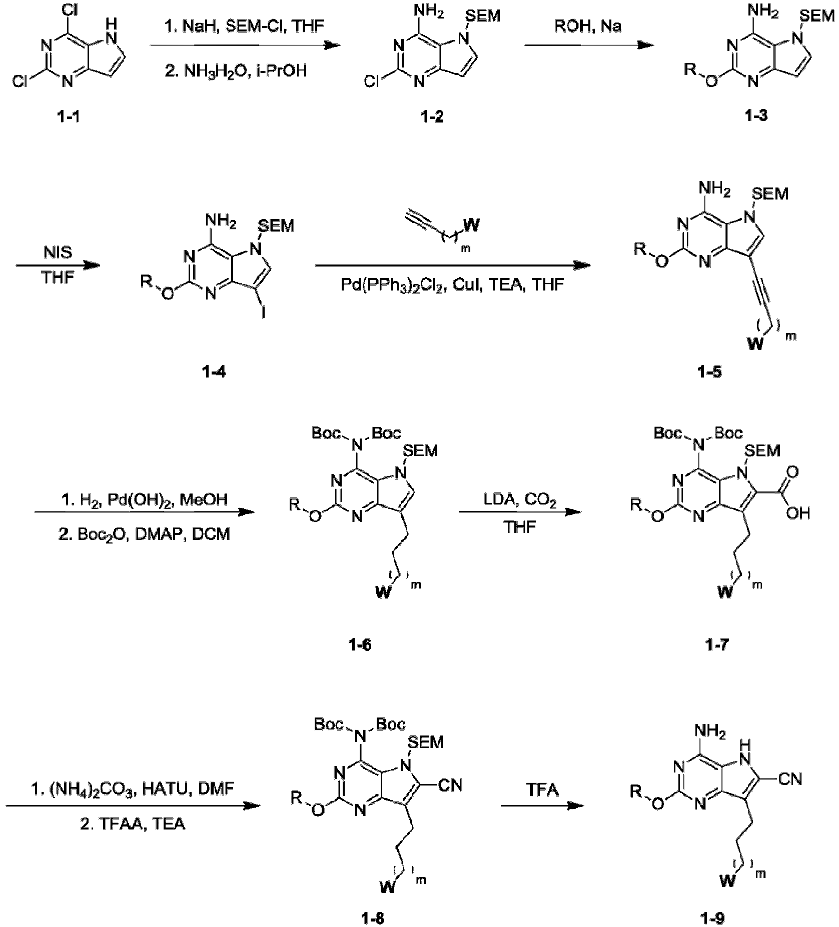

Commercially available starting material 2,4-dichloro-5*H*-pyrrolo[3,2-*d*]pyrimidine (**1-1**) was first reacted with SEM-Cl to substitute the proton on nitrogen with SEM protecting group, and reacted with aqueous ammonia at a higher temperature (90-100 °C) to afford 2-chloro-5-((2-(trimethylsilyl)ethoxy)methyl)-5*H*-pyrrolo[3,2-*d*]pyrimidin-4-amine (**1-2**); sodium alkoxide, prepared by different types of alcohols (represented by ROH) and sodium metal, was reacted with compound **1-2** in corresponding alcohol (e.g. *n*-butanol) to afford alkoxy substituted compound **1-3**; **1-3** was iodinated by NIS to afford compound **1-4**; alkynyl compound (**1-5**) was prepared by the iodinated compound and different types of terminal alkynes *via* Pd-catalyzed coupling reaction; then, **1-5** was hydrogenated catalyzed by Pd(OH)₂/C and amino group was protected by Boc to afford compound (**1-6**); compound **1-6** with high purity was reacted with LDA at -78 °C for a certain time, such as 1 h, followed by being treated with CO₂ and acidified cautiously to afford carboxyl substituted compound (**1-7**); the carboxyl of **1-7** was transformed to amide by ammonium carbonate *via* amide coupling reaction and dehydrated in trifluoroacetic anhydride/triethylamine to afford compound **1-8**; followed by simple work-up, SEM protecting group was removed in trifluoroacetic acid at room temperature to afford target product (**1-9**).

### General route 2:

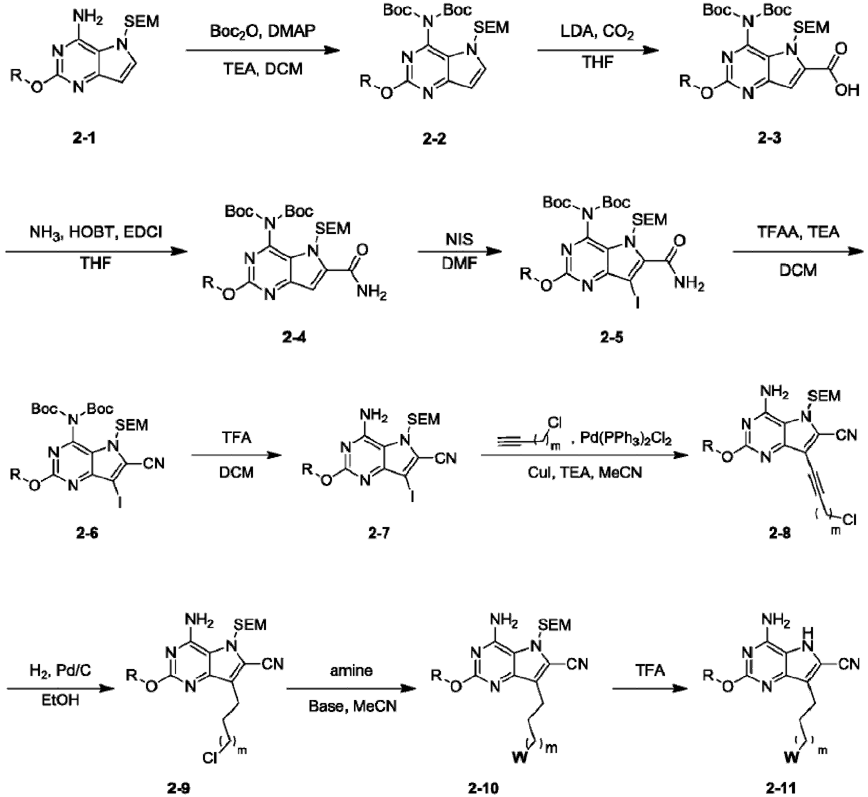

Free amino group of compound **2-1** was protected by Boc, and *di*-Boc protected compound **2-2** was obtained after slurring; this compound was reacted with LDA at low temperature, continuously purged with dry CO₂ and acidified to afford carboxyl substituted compound **2-3**; crude **2-3** was transformed to compound **2-4** *via* amidation, which was purified by slurring for several times, and followed by being iodinated by NIS to afford compound **2-5**; **2-5** was dehydrated in trifluoroacetic anhydride/triethylamine to afford cyanide substituted compound **2-6**; crude **2-6** was treated by diluted trifluoroacetic acid in dichloromethane solution to deprotect Boc to afford intermediate **2-7**; this compound was reacted with chlorinated aliphatic terminal alkyne *via* Sonogoshira cross-coupling to afford compound **2-8**; **2-8** was catalytically hydrogenated to afford intermediate **2-9**; this intermediate alkylated different types of amine reagents, and afforded target product **2-11** after the deprotection of SEM protecting group by trifluoroacetic acid and prep-HPLC purification.

### General route 3:

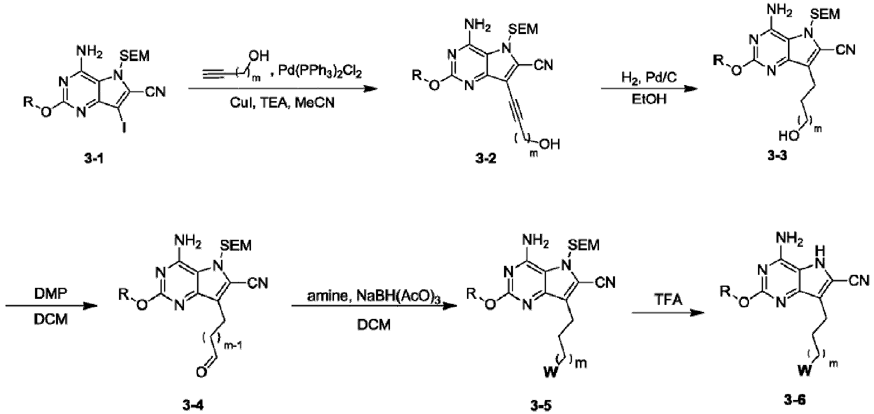

Compound **3-1** was reacted with aliphatic terminal-alkynyl alcohol *via* Sonogoshira cross-coupling to afford compound **3-2**; **3-2** was catalytically hydrogenated to afford corresponding saturated alcohol (**3-3**); compound **3-3** was oxidized by DMP to afford corresponding aldehyde **3-4**; followed by reacting with different types of amine reagents *via* reductive amination to afford compound **3-5**; SEM protecting group of this compound was removed by trifluoroacetic acid to afford target product **3-6** after prep-HPLC purification.

### General route 4:

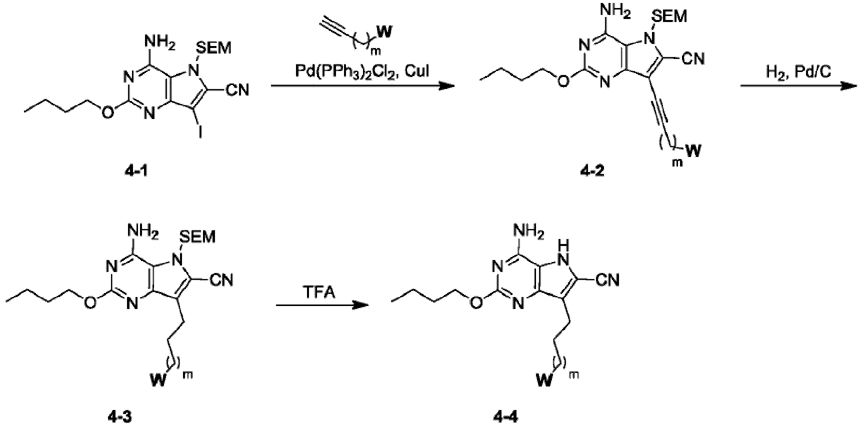

4-Amino-2-butoxy-7-iodo-5-((2-(trimethylsilyl)ethoxy)methyl)pyrrolo[3,2-*d*]pyrimidi ne-6-carbonitrile (**4-1**) was cross-coupled with terminal alkyne to afford 7-alkynyl substituted compound (**4-2**), and target product (**4-4**) was obtained after catalytic hydrogenation and SEM deprotection.

It will be appreciated by those skilled in the art that in order to prepare the compounds of the present invention, the order of the reaction steps in the reaction routes may be different, which is also within the scope of the present invention.

For the sake of clarity, the present invention is further illustrated by the examples. But the embodiments are not limited to the definition or designation of the scope of the invention.

### Preparation of examples 1 to 3 according to general route 1

### Example 1

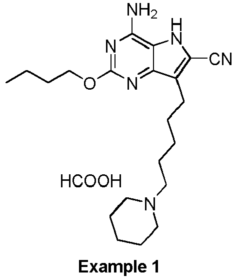

### 4-Amino-2-butoxy-7-(5-(piperidin-1-yl)pentyl)-5H-pyrrolo[3,2-d]pyrimidine-6-carbonitrile formate

### Reaction route: Preparation of Example 1

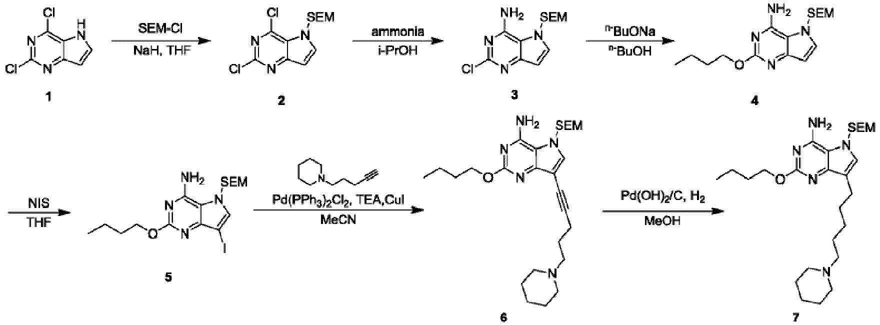

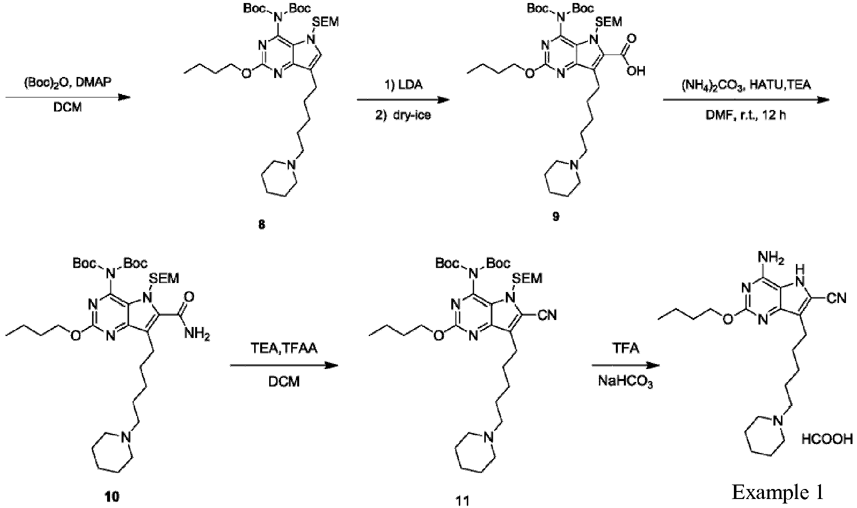

### Step A: synthesis of 2,4-dichloro-5-((2-trimethylsilyl)ethoxy)methyl)pyrrolo [3,2-d]pyrimidine

2,4-Dichloro-5*H*-pyrrolo[3,2-*d*]pyrimidine (4.0g, 21.39mmol) was dissolved in anhydrous tetrahydrofuran (30mL). Sodium hydride (w/w 60%, 1.03g, 25.75mmol) was added portionwise at 0°C under nitrogen atmosphere. The mixture was stirred at ambient temperature for 30 minutes and cooled to 0 °C again, then (2-(chloromethoxy)ethyl)trimethylsilane (3.9g, 23.49mmol) was added dropwise. After the addition, this reaction mixture was stirred at ambient temperature for further 2 hours, quenched with water (120mL) and extracted with ethyl acetate (100mL × 2). The combined organic layer was washed with saturated sodium carbonate solution and brine, dried over anhydrous sodium sulfate and concentrated under vacuum. The residue was purified by column chromatograph on silica gel (ethyl acetate/petroleum ether 5% to 10%) to afford 2,4-dichloro-5-((2-(trimethylsilyl) ethoxy)methyl)pyrrolo[3,2-*d*]pyrimidine (5.8g, 85.55% yield) as a yellow solid. MS (ESI) m/z: 318 [M+H⁺].

### Step B: synthesis of 2-chloro-5-((2-(trimethylsilyl)ethoxy)methyl)pyrrolo[3,2-d] pyrimidine-4-amine

To a 1000mL autoclave, were added 2,4-dichloro-5-((2-(trimethylsilyl) ethoxy)methyl)pyrrolo[3,2-*d*]pyrimidine (5g, 15.77mmol), isopropanol (15mL) and aqueous ammonia (250mL) sequentially. The reaction mixture was stirred at 100-110°C for 3 hours. After cooling to ambient temperature, this mixture was diluted with water (250mL) and filtered to obtain the crude of 2-chloro-5-((2-(trimethylsilyl)ethoxy)methyl)pyrrolo[3,2-*d*]pyrimidin-4-amine (4.0g) as white solid which was used for the next step directly. MS (ESI) m/z: 299 [M+H⁺] .

### Step C: synthesis of 4-amino-2-butoxy-5-((2-(trimethylsilyl)ethoxy)methyl pyrrolo[3,2-d]pyrimidine

Sodium (1.24g, 53.91mmol) was added to anhydrous butanol (55mL) under nitrogen atmosphere, the mixture was stirred at room temperature until sodium was dissolved completely, 4-amino-2-chloro-5-((2-(trimethylsilyl)ethoxy)methylpyrrolo[3,2-*d*] pyrimidine (4.0g, 13.42mmol) was then added. The reaction mixture was stirred for 8 hours at 100°C. After cooling to ambient temperature, this mixture was poured to water (200mL) slowly and extracted with ethyl acetate (100mL × 3). The combined organic layer was washed with brine, dried over anhydrous sodium sulfate and filtered, the filtrate was concentrated under vacuum. The residue was purified by column chromatograph on silica gel (gradient elution: ethyl acetate/petroleum ether 15% to 25%) to afford 4-amino-2-butoxy-5-((2-(trimethylsilyl)ethoxy)methyl pyrrolo[3,2-*d*]pyrimidine (4.1g, 90.91% yield) as a yellow solid. MS (ESI) m/z: 337 [M+H⁺] .

### Step D: synthesis of 4-amino-2-butoxy-7-iodo-5-((2-(trimethylsilyl)ethoxy)methyl) pyrrolo[3,2-d]pyrimidine

To a solution of 4-amino-2-butoxy-5-((2-trimethylsilyl)ethoxy)methyl)pyrrolo [3,2-*d*]pyrimidine (40.00g, 119.0mmol) in tetrahydrofuran (500mL) was added NIS (32.13g, 142.8mmol) in three portions at 0°C under nitrogen atmosphere. After the addition, the mixture was stirred at 30°C for 2 hours. TLC showed the reagent was completely reacted. The reaction mixture was poured into saturated aqueous sodium thiosulfate (1000mL) and stirred for 10 minutes. The aqueous phase was extracted with ethyl acetate (300mL × 2). The combined organic phase was washed with saturated brine (200mL × 2), dried over anhydrous sodium sulfate, filtered and the filtrate was concentrated in vacuum to afford 4-amino-2-butoxy-7-iodo-5-((2-trimethylsilyl)ethoxy)methyl)pyrrolo[3,2-*d*]pyrimidin e (40.00g, 72.70% yield) as yellow solid, which was used for the next step directly. **¹H** NMR (400 MHz, CHLOROFORM-*d*) δ 7.22 (s, 1H), 5.95 (br. s., 2H), 5.45-5.37 (m, 2H), 4.48-4.36 (m, 2H), 3.67-3.56 (m, 2H), 1.86-1.75 (m, 2H), 1.53 (t, *J =* 7.4 Hz, 2H), 1.06-0.92 (m, 5H), 0.01 (s, 9H).

### Step E: synthesis of 4-amino-2-butoxy-7-[5-(1-piperidyl)pent-1-ynyl]-5-((2-(trimethylsilyl)ethoxy)methyl)pyrrolo[3,2-d]pyrimidine

To a three-necked flask, were added 4-amino-2-butoxy-7-iodo-5-((2-(trimethylsilyl)ethoxy)methyl)pyrrolo[3,2-*d*]pyrimidi ne (4.00g, 8.65mmol), 5-(1-piperidyl)-1-pentyne (1.57g, 10.38mmol), bis(triphenylphosphine)palladium (II) dichloride (607mg, 865.00µmol) and triethylamine (2.63g, 25.95mmol) in acetonitrile (60mL). The reaction mixture was stirred at 30°C for 12 hours after swept by nitrogen gas for 3 times. The reaction mixture was filtered and the filtrate was concentrated in vacuum. The residue was purified by silica gel chromatography (column height: 250mm, diameter: 100mm, 100-200mesh silica gel, petroleum ether/ethyl acetate = 3/1, 0/1) to afford 4-amino-2-butoxy-7-[5-(1-piperidyl)pent-1-ynyl]-5-((2-trimethylsilyl)ethoxy)methyl) pyrrolo[3,2-*d*]pyrimidine (2.00g, 47.60% yield). MS (ESI) m/z: 486 [M+H⁺].

### Step F: synthesis of 4-amino-2-butoxy-7-(5-(1-piperidyl)pentyl]-5-((2-trimethylsilyl) ethoxy)methyl)pyrrolo[3,2-d]pyrimidine

4-amino-2-butoxy-7-[5-(1-piperidyl)pent-1-ynyl]-5-(2-trimethylsilyl)ethoxy)methyl)p yrrolo[3,2-*d*]pyrimidine (2.10g, 4.32mmol) was dissolved in methanol (30mL), palladium hydroxide/carbon (w/w: 10%, 0.20g) was added under nitrogen atmosphere. The reaction mixture was purged with hydrogen gas for 5 times and stirred under hydrogen atmosphere (50psi) at 30°C for 48 hours. LC-MS showed the starting material was consumed completely. The reaction mixture was filtered and the cake was washed with methanol repeated, the filtrate was combined and concentrated under vacuum to give 4-amino-2-butoxy-7-(5-(1-piperidyl)pentyl]-5-((2-trimethylsilyl) ethoxy)methyl)pyrrolo[3,2-*d*]pyrimidine (1.80g, 85.43% yield) as brown oil which was used directly for the next step. MS (ESI) m/z: 490 [M+H⁺].

### Step G: synthesis of 4-(bis(tert-butoxycarbonyl)amino)-2-butoxy-7-(5-(1-piperidyl) pentyl)-5-((2-(trimethylsilyl)ethoxy)methyl)pyrrolo[3,2-d]pyrimidine

4-Amino-2-butoxy-7-(5-(1-piperidyl)pentyl]-5-((2-(trimethylsilyl)ethoxy)methyl)pyrr olo[3,2-*d*]pyrimidine (798.33mg, 1.63mmol) and triethylamine (494.82mg, 4.89mmol) were dissolved in anhydrous dichloromethane (20mL), di-*tert*-butyl pyrocarbonate (1.07g, 4.89mmol) was added portionwise. After the addition, the reaction mixture was stirred at 30°C under nitrogen atmosphere for 12 hours. TLC showed that the reaction was complete, then the reaction mixture was concentrated under reduced pressure. The residue was purified by silica gel chromatography (column height: 250mm, diameter: 100mm, 100-200 mesh silica gel, dichloromethane/methanol = 100/1) to afford 4-(bis(*tert*-butoxycarbonyl)amino)-2-butoxy-7-(5-(1-piperidyl) pentyl)-5-((2-(trimethylsilyl)ethoxy)methyl)pyrrolo[3,2-*d*]pyrimidine (600.00mag, 53.35% yield) as yellow oil which was used for the next step directly.

### Step H: synthesis of 4-(bis(tert-butoxycarbonyl)amino)-2-butoxy-7-(5-(1-piperidyl) pentyl)-5-((2-(trimethylsilyl)ethoxy)methyl)pyrrolo[3,2-d]pyrimidine-6-carboxylic acid formate

To a three-necked flask, were added 4-(bis(*tert*-butoxycarbonyl)amino)-2-butoxy-7-(5-(1-piperidyl)pentyl)-5-((2-(trimethy lsilyl)ethoxy)methyl)pyrrolo[3,2-*d*]pyrimidine (400.00mg, 579.71µmol) and tetrahydrofuran (10mL) The solution was cooled to -70 °C , LDA (1M in tetrahydrofuran, 1.74mL) was added dropwise. The mixture was stirred at -70 °C for 1 hour, then poured into dry-ice quickly and airproofing with balloon. The reaction mixture was stirred for further 30 minutes and warmed to room temperature. The mixture was quenched by saturated aqueous ammonium chloride, extracted with ethyl acetate (30mL × 3). The organic phase was washed with saturated brine (30mL), dried over anhydrous sodium sulfate and filtered, the filtrate was concentrated under vacuum to give a residue, which was purified by preparative HPLC (FA condition) to afford 4-[bis(*tert*-butoxycarbonyl)amino]-2-butoxy-7-(5-(1-piperidyl)pentyl)-5-((2-(trimethylsilyl)ethoxy)methyl)pyrrolo[3,2-*d*]pyrimidine-6-carboxylic acid formate (100.00mg, 22.11% yield). MS (ESI) m/z: 734 [M+H⁺].

### Step I: synthesis of 4-(bis(tert-butoxycarbonyl)amino)-2-butoxy-7-(5-(1-piperidyl)pentyl)-5-((2-(trimethylsilyl)ethoxy)methyl)pyrrolo[3,2-d]pyrimidine-6-f ormamide

To a solution of 4-(bis(*tert*-butoxycarbonyl)amino)-2-butoxy-7-(5-(1-piperidyl)pentyl)-5-((2-(trimethylsilyl)ethoxy)methyl)pyrrolo[3,2-*d*]pyrimidine-6-c arboxylic acid (80.00mg, 108.99µmol) in DMF (2mL), were added ammonium carbonate (104.73mg, 1.09mmol), triethylamine (27.57mg, 272.48µmol) and HATU (62.16mg, 163.49µmol) sequentially. The mixture was stirred at 30°C for 12 hours under nitrogen atmosphere, then poured into water (20mL) and extracted with ethyl acetate (20mL × 3). The combined organic phase was washed with saturated brine (20mL), dried over anhydrous sodium sulfate and filtered, the filtrate was concentrated under vacuum. The residue was purified by preparative TLC (dichloromethane/methanol=5/1) to afford 4-(bis(*tert*-butoxycarbonyl)amino)-2-butoxy-7-(5-(1-piperidyl)pentyl)-5-((2-(trimethylsilyl)ethoxy)methyl)pyrrolo[3,2-*d*]p yrimidine-6-formamide (60mg, crude). MS (ESI) m/z: 733 [M+H⁺].

### Step J: synthesis of 4-(bis(tert-butoxycarbonyl)amino)-2-butoxy-7-(5-(1-piperidyl)pentyl)-5-((2-(trimethylsilyl)ethoxy)methyl)pyrrolo[3,2-d]pyrimidine-6-n itrile

To a three-necked flask, were added 4-(bis(*tert*-butoxycarbonyl)amino)-2-butoxy-7-(5-(1-piperidyl)pentyl)-5-((2-(trimethylsilyl)ethoxy)methyl)pyrrolo[3,2-*d*]p yrimidine-6-formamide (60.00mg, 81.85µmol), triethylamine (372.72mg, 3.68mmol) and dichloromethane (2mL) sequentially. Trifluoroacetic anhydride was added (343.83mg, 1.64mmol) dropwise at 0°C . After the addition, the mixture was stirred at 30 °C for 12 hours, then diluted with water (20mL) and extracted with dichloromethane (10mL × 3). The combined organic phase was washed with saturated brine (10mL), dried over anhydrous sodium sulfate and filtered, the filtrate was concentrated under vacuum to afford 4-(bis(*tert*-butoxycarbonyl)amino)-2-butoxy-7-(5-(1-piperidyl)pentyl)-5-((2-(trimethy lsilyl)ethoxy)methyl)pyrrolo[3,2-*d*]pyrimidine-6-nitrile (60.00mg, crude) as light yellow solid. MS (ESI) m/z: 715 [M+H⁺].

### Step K: synthesisi of 4-amino-2-butoxy-7-(5-(1-piperidyl)pentyl)-5H-pyrrolo[3,2-d] pyrimidine-6-nitrile formate

4-(Bis(*tert*-butoxycarbonyl)amino)-2-butoxy-7-(5-(1-piperidyl)pentyl)-5-((2-(trimethy lsilyl)ethoxy)methyl)pyrrolo[3,2-*d*]pyrimidine-6-nitrile (60.00mg, 83.91µmol) was dissolved in trifluoroacetic acid (2.00mL), the mixture was stirred at 30°C for 5 hour under nitrogen atmosphere. Trifluoroacetic acid was removed by concentration under vacuum. The residue was basified by saturated sodium bicarbonate aqueous solution until pH was around 8, the reaction mixture was extracted with ethyl acetate (10mL×3). The organic phase was dried over anhydrous sodium sulfate and concentrated under reduced pressure. The residue was purified by preparative HPLC (FA condition) to give 4-amino-2-butoxy-7-(5-(1-piperidyl)pentyl) -5*H*-pyrrolo[3,2-*d*]pyrimidine-6-carbonitrile formate (19.00mg) as a white solid. **¹H** NMR (400 MHz, METHANOL-*d₄*) δ 8.18 (s, 1H), 4.38 (t, *J* = 6.5 Hz, 2H), 3.65-3.42 (m, 3H), 3.12-3.00 (m, 2H), 2.85 (t, *J* = 7.3 Hz, 3H), 2.03-1.70 (m, 12H), 1.63-1.37 (m, 4H), 1.01 (t, *J* = 7.4 Hz, 3H). MS (ESI) m/z: 385 [M+H⁺].

### Example: Preparation of 1-(pent-1'-ynyl)piperidine

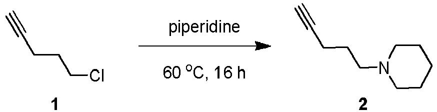

A solution of 5-chloro-1-pentyne (4.00g, 39.00mmol) in piperidine (16.60g, 195.01mmol) was stirred at 60°C for 12 hours. TLC showed the reaction was complete. The reaction mixture was cooled to room temperature slowly and poured into water (30mL) and stirred for 10 min. The aqueous phase was extracted with ethyl acetate (30mL × 3). The combined organic phase was washed with water (30mL × 3), dried over anhydrous sodium sulfate, filtered and the filtrate was concentrated under vacuum to give 5-(1-piperidyl)-1-pentyne (5.20g, 88.15% yield) as yellow liquid. **¹H** NMR (400 MHz, CHLOROFORM-*d*) δ 2.42-2.29 (m, 6H), 2.19 (dt, *J* = 2.6, 7.2 Hz, 2H), 1.92 (t, *J* = 2.6 Hz, 1H), 1.73-1.66 (m, 2H), 1.58-1.53 (m, 4H), 1.44-1.37 (m, 2H).

### Example 2

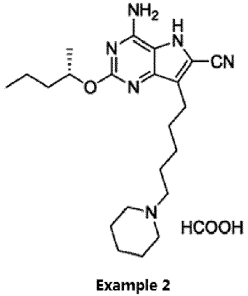

### (S)-4-amino-(1-methylbutoxy)-7-(5-(piperidin-1-yl)pentyl)-5H-pyrrolo[3,2-d]pyrimid ine-6-carbonitrile formate

### Step A: synthesis of 4-(bis(tert-butoxycarbonyl)amino)-2-((S)-1-methylbutoxy)-7-(5-(1-piperidyl)pentyl)-5-((2-(trimethylsilyl)ethoxy)methyl)pyrrolo[3,2-d]pyrimidine-6-n itrile

4-(Bis(*tert*-butoxycarbonyl)amino)-2-((S)-1-methylbutoxy)-7-(5-(1-piperidyl)pentyl)-5-((2-(trimethylsilyl)ethoxy)methyl)pyrrolo[3,2-*d*]pyrimidine-6-nitrile was prepared according to Steps C, D, E, F, G, H, I, J of the process in Example 1 except for replacing the butanol with (S)-1-methyl butanol.

### Step B: synthesis of 4-amino-2-((S)-1-methylbutoxy)-7-(5-(1-piperidyl)pentyl)-5-((2-(trimethylsilyl)ethoxy)methyl)pyrrolo[3,2-d]pyrimidin-6-nitrile

4-(Bis(*tert*-butoxycarbonyl)amino)-2-((S)-1-methylbutoxy)-7-(5-(1-piperidyl)pentyl)-5-((2-(trimethylsilyl)ethoxy)methyl)pyrrolo[3,2-*d*]pyrimidine-6-nitrile (30.00mg, 54.87µmol) was dissolved in dichloromethane (2mL) and trifluoroacetic acid (0.5mL) was added. The mixture was stirred at room temperature for 12 hours. LC-MS showed the reaction was complete. The reaction mixture was slowly poured into saturated sodium bicarbonate aqueous solution (30mL) and extracted with dichloromethane (20mL × 3). The combined organic layer was washed with saturated brine (5mL × 2), dried over anhydrous sodium sulfate, filtered and the filtrate was concentrated under reduced pressure to give 4-amino-2-((*S*)-1-methylbutoxy)-7-(5-(1-piperidyl)pentyl)-5-((2-(trimethylsilyl)ethox y)methyl)pyrrolo[3,2-*d*]pyrimidine-6-carbonitrile (30mg, crude) which was used for next step directly. MS (ESI) m/z: 529.4 [M+H⁺].

### Step C: synthesis of 4-amino-2-((S)-1-methylbutoxy)-7-(5-(1-piperidyl)pentyl)-5H-pyrrolo[3,2-d]pyrimidine-6-carbonitrile formate

4-Amino-2-((*S*)-1-methylbutoxy)-7-(5-(1-piperidyl)pentyl)-5-((2-(trimethylsilyl)ethox y)methyl)pyrrolo[3,2-*d*]pyrimidine-6-carbonitrile (30.00mg, 56.73µmol) was dissolved in THF (2mL), tetrabutylammonium fluoride (0.13mL, 1M in tetrahydrofuran) was added in one portion. The mixture was stirred at 60 °C for 12 hour. LC-MS showed that the reaction was complete, the mixture was cooled to room temperature naturally and then slowly poured into saturated sodium bicarbonate aqueous solution (10mL), and then extracted with ethyl acetate (10mL × 3). The combined organic layer was washed with saturated brine (5mL × 2), dried over anhydrous sodium sulfate, filtered and the filtrate was concentrated under reduced pressure. The residue was purified by prep-HPLC (FA condition) to obtain 4-amino-2-((*S*)-1-methylbutoxy)-7-(5-(1-piperidyl)pentyl)-5*H*-pyrrolo[3,2-*d*]pyrimidi ne-6-carbonitrile formate (3.80mg, 16.81% yield) as a white solid. **¹H** NMR (400 MHz, METHANOL-*d₄*): δ 8.56 (br. s., 1H), 5.23 (dd, *J* = 6.27, 12.55 Hz, 1H), 2.89-3.22 (m, 6H), 2.84 (t, *J* = 7.28 Hz, 2H), 1.38-1.95 (m, 16H), 1.33 (d, *J* = 6.02 Hz, 3H), 0.97 (t*, J* = 7.15 Hz, 3H). MS (ESI) m/z: 399.3 [M+H⁺].

### Example 3

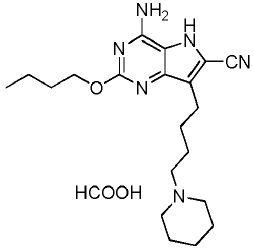

### Example 3

### 4-amino-2-butoxy-7-(4-(piperidin-1-yl)butyl)-5H-pyrrolo[3,2-d]pyrimidine-6-carboni trile formate

4-Amino-2-butoxy-7-(4-(piperidin-1-yl)butyl)-5*H-*pyrrolo[3,2-*d*]pyrimidine-6-carbon itrile formate was prepared according to steps E, F, G, H, I, J, K of the process in example 1, except for replacing 5-(1-piperidyl)-1-pentyne with 4-(1-piperidyl)-1-butyne. **¹H** NMR (400 MHz, METHANOL-*d₄*): δ 8.49 (brs., 1H), 4.34 (t, *J* = 6.5 Hz, 2H), 3.32-2.65 (m, 8H), 1.96- 1.45 (m, 14H), 1.01 (t, *J* = 7.4 Hz, 3H).

### Example: Preparation of 4-(1-piperidyl)-1-butyne

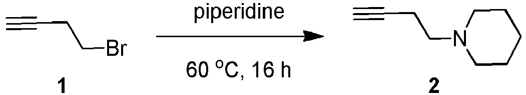

4-Bromo-1-butyne(2.00g 15.04mmol) was dissolved in acetone (2mL), then the mixture was cooled to 0°C, piperidine (1.28g, 15.04mmol) and cesium carbonate (4.90g, 15.04mmol) were added sequentially. The reaction mixture was stirred under 0°C for 5 min and then warmed to room temperature slowly, further stirred for 2 hours. The reaction mixture was poured into water (20mL), and extracted with dichloromethane (20mL × 2). The combined organic layer was washed with water (30mL × 3), dried over anhydrous sodium sulfate. After filtration, and the filtrate was concentrated under vacuum to give 4-(1-piperidyl)-1-butyne (600.00mg, 4.37mmol, yield: 29.07%) as brown liquid. **¹H** NMR (400 MHz, CHLOROFORM-*d*): δ 2.63-2.56 (m, 2H), 2.49-2.37 (m, 6H), 1.99 (t, *J* = 2.7 Hz, 1H), 1.61 (m, 4H), 1.50-1.41 (m, 2H).

### Preparation of examples 4 to 19 according to general route 2

### Example 4

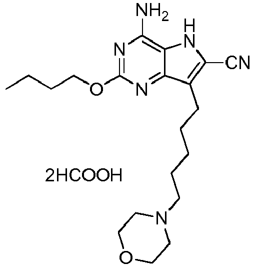

### Example 4

### 4-amino-2-butoxy-7-(5-morpholin-1-yl)pentyl)-5H-pyrrolo[3,2-d]pyrimidine-6-carbonitrile formate

### Route: Preparation of Example 4

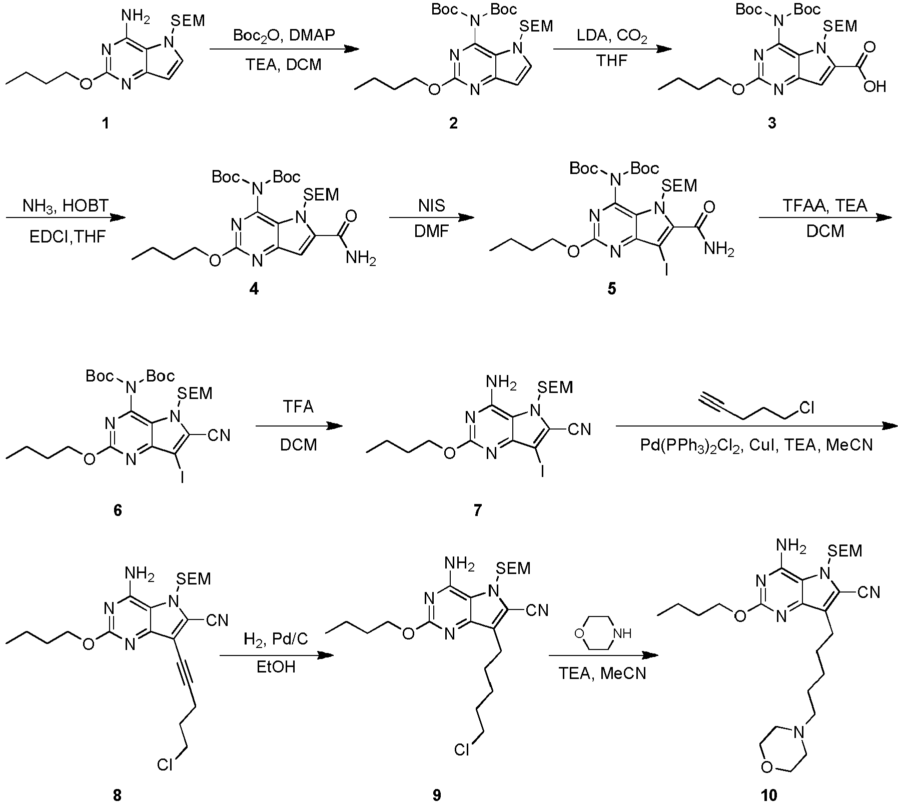

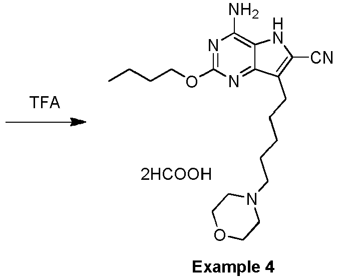

### Step A : synthesis of 4-((bis-tert-butoxycarbonyl)-amino)-2-butoxy-5-((2-(trimethylsilyl)ethoxy)methyl)pyrrolo[3,2-d]pyrimidine

4-Amino-2-butoxy-5-((2-(trimethylsilyl)ethoxy)methyl)pyrrolo[3,2-*d*]pyrimidine (50g, 148.59mmol) was dissoved in anhydrous tetrahydrofuran (300mL), triethylamine (16.54g, 163.45mmol) and *N,N-*dimethylpyridin-4-amine (907.65mg, 7.43mmol) were added sequentially while stirring. Di-tert-butyl dicarbonate (113.50g, 0.52mol) was dissolved in additional anhydrous tetrahydrofuran (200mL) while the temperature was maintained at around 20°C, and the resultant solution was slowly added to the aforementioned reaction solution dropwise. After the addition, the reaction mixture was stirred at around 20 °C for 16 hours. The reaction was complete, monitored by LC-MS. The reaction mixture was poured into saturated aqueous sodium bicarbonate solution (500mL), and extracted with ethyl acetate (500mL). The organic phase was washed with saturated aqueous ammonium chloride (500mL) and water (500mL) sequentially, and dried over anhydrous sodium sulfate. After filtration, the filtrate was concentrated under vacuum. The resultant solid was added to hexane (300mL) and the suspension was stirred vigorously for 1 hour and then filtered. The filter cake was dried under reduced pressure to give 4-((bis-*tert-*butoxycarbonyl)-amino)-2-butoxy-5-((2-(trimethylsilyl)ethoxy)methyl)py rrolo[3,2-*d*]pyrimidine (67.00g, 124.83mmol, yield: 84.01%) as a white powder. **¹H** NMR (400 MHz, CHLOROFORM-*d*): δ 7.46 (d, *J* = 3.1 Hz, 1H), 6.55 (d, *J* = 3.3 Hz, 1H), 5.38 (s, 2H), 4.38 (t, *J* = 6.6 Hz, 2H), 3.34-3.46 (m, 2H), 1.75-1.89 (m, 2H), 1.47-1.58 (m, 2H), 1.41 (s, 18H), 0.97 (t, *J* = 7.4 Hz, 3H), 0.88 (dd, *J* = 9.0, 7.7 Hz, 2H), 0.00 (s, 9H).

### Step B: synthesis of 4-((bis-tert-butoxycarbonyl)-amino)-2-butoxy-5-((2-(trimethylsilyl)ethoxy)methyl)pyrrolo[3,2-d]pyrimidine-6-carboxylic acid

4-((Bis-*tert*-butoxycarbonyl)-amino)-2-butoxy-5-((2-(trimethylsilyl)ethoxy)methyl)py rrolo[3,2-*d*]pyrimidine (87.00g, 162.09mmol) was dissolved in anhydrous tetrahydrofuran (870mL), the mixture was cooled to -65 °C under nitrogen atmosphere. The intertemperature of the reaction mixture was controlled at or lower than -60°C, a solution of 2M LDA (121.57mL, 243.14mmol) in heaxane was dropwise added. After the addition, the reaction mixture was stirred at for 1 hour. Then, similarly, the reaction mixture was controlled at -60 °C or below and dryed carbon dioxide gas was bublled slowly while stirring until complete consumption of starting materal was monitored by LC-MS (around 2 hours). The reaction was quenced by slow addition of saturated aqueous ammonium chloride solution (800mL) and then warmed to room temperature. The mxiture was extracted with ethyl acetate (800mL), and the organic layer was washed with saturated aqueous ammonium chloride solution (800mL) twice and saturated brine (500mL) once, dried over anhydrous sodium sulfate. After filtration, and the filtrate was concentrated under reduced pressure to give 4-((bis-*tert*-butoxycarbonyl)-amino)-2-butoxy-5-((2-(trimethylsilyl)ethoxy)methyl)pyrrolo[3,2-*d*]pyrimidine-6-carboxylic acid (94.13g, crude) as red oil, which was used for the next step directly. MS (m/z): 581 [M+H⁺].

### Step C : synthesis of 4-((bis-tert-butoxycarbonyl)-amino)-2-butoxy-5-((2-(trimethylsilyl)ethoxy)methyl)pyrrolo[3,2-d]pyrimidine-6-formamide

The crude product of 4-((bis-*tert-*butoxycarbonyl)-amino)-2-butoxy-5-((2-(trimethylsilyl)ethoxy)methyl)pyrrolo[3,2-*d*]pyrimidine-6-carboxylic acid (94.13g, obtained from above step) was dissolved in tetrahydrofuran (600mL). HOBt (21.90g, 162.09mmol) and EDCI (31.07g, 162.09mmol) were added sequencially at room temperature. The mixture was stirred at 30-40 °C for 1 hour. Ammonia was purged to tetrahydrofuran until the solution was saturated at 0°C, the ammonia solution (250mL) was slowly added to aforementioned mixture dropwise at 30-40°C. After the addition, the reaction mixture was stirred at 30-40°C for 16 hours. The reaction was complete, monitored by LC-MS. The reaction was quenced by an addition of saturated aqueous ammonium chloride solution (800mL) and extracted with ethyl acetate (800mL). The organic layer was washed with saturated aqueous ammonium chloride solution (800mL × 2) and saturated brine (500mL) once, dried over anhydrous sodium sulfate. After filtration, and the filtrate was concentrated under reduced pressure to give a tan solid residue. To the residue, was added hexane/ethyl acetate (10/1, 150mL) and the suspension was stirred vigorously for 1 hour and then filtered. The filter cake was dried under a reduced pressure to give 4-((bis-*tert-*butoxycarbonyl)-amino)-2-butoxy-5-((2-(trimethylsilyl)ethoxy)methyl)py rrolo[3,2-*d*]pyrimidine-6-formamide (55.80g, 96.25mmol, yield for 2 steps: 59.38 %) as a yellow powder. **¹H** NMR (400 MHz, CHLOROFORM-*d*): δ 6.94 (s, 1H), 5.81 (s, 2H), 4.39 (t, *J* = 6.6 Hz, 2H), 3.43-3.56 (m, 2H), 1.79-1.88 (m, 2H), 1.48-1.57 (m, 2H), 1.42 (s, 18H), 0.98 (t, *J* = 7.4 Hz, 3H), 0.85-0.92 (m, 2H), 0.00 (s, 9H).

### Step D: synthesis of 4-((bis-tert-butoxycarbonyl)-amino)-2-butoxy-7-iodo-5-((2-(trimethylsilyl)ethoxy)methyl)pyrrolo[3,2-d]pyrimidine-6-formamide

4-((Bis-*tert*-butoxycarbonyl)-amino)-2-butoxy-5-((2-(trimethylsilyl)ethoxy)methyl)py rrolo[3,2-*d*]pyrimidine-6-formamide (55.80g, 96.25mmol) was dissolved in *N,N-*dimethylformamide (560mL), *N*-iodosuccinimide (43.31g, 192.50mmol) was added portionwise at room temperature while stirring, and the reaction mixture was heated to 60-70°C and stirred for 72 hours after the addition was complete. After the reaction was completed as monitored by LC-MS, the reaction mixture was cooled to room temperature, and slowly poured into saturated aqueous sodium thiosulfate solution (1500mL) under stirring, and extracted with ethyl acetate (500mL). The organic layer was washed with aqueous sodium hydroxide solution (0.5M, 500mL) twice and saturated brine (300mL) once, then dried over anhydrous sodium sulfate. After filtration, the filtrate was concentrated under reduced pressure to give a tan solid residue. The solid residue was added to hexane/ethyl acetate (15/1, 100mL) and the suspension was stirred vigorously for 1 hour and then filtered. The filter cake was dried under reduced pressure to give 4-((bis-*tert*-butoxycarbonyl)-amino)-2-butoxy-7-iodo-5-((2-(trimethylsilyl)ethoxy)me thyl)pyrrolo[3,2-*d*]pyrimidine-6-formamide (51.00g, 72.27mmol, yield: 75.09%) as a tan powder. **¹H** NMR (400 MHz, CHLOROFORM-*d*) δ: 6.60 (br, s., 1H), 6.14 br. s., 1H), 5.71 (s, 2H), 4.46 (t, *J* = 6.6 Hz, 2H), 3.43-3.54 (m, 2H), 1.78-1.90 (m, 2H), 1.53 (d, *J* = 7.5 Hz, 2H), 1.41 (s, 18H), 0.99 (t, *J* = 7.3 Hz, 3H), 0.81-0.93 (m, 2H), 0.00 (s, 9H).

### Step E: synthesis of 4-((bis-tert-butoxycarbonyl)-amino)-2-butoxy-7-iodo-5-((2-(trimethylsilyl)ethoxy)methyl)pyrrolo[3,2-d]pyrimidine-6-nitrile

4-((Bis-*tert*-butoxycarbonyl)-amino)-2-butoxy-7-iodo-5-((2-(trimethylsilyl)ethoxy)me thyl)pyrrolo[3,2-*d*]pyrimidine-6-formamide (51.00g, 72.27mmol) was dissolved in anhydrous dichloromethane (500mL), triethylamine (36.57g, 361.35mmol) was added in one portion. Under nitrogen atmosphere, the reaction mixture was cooled to 0°C and thereto was slowly added trifluoroacetic anhydride (37.95g, 180.68mmol) dropwise over a period of 0.5 hour. After the addition, the reaction mixture was warmed to 20°C and stirred for 16 hours. After the reaction solution was complete as monitored by LC-MS, it was washed with saturated aqueous sodium bicarbonate solution (500mL×2), saturated aqueous ammonium chloride solution (500mL×2), saturated brine once, and dried over anhydrous sodium sulfate and filtered. The filtrate was concentrated under reduced pressure to give 4-((bis-*tert-*butoxycarbonyl)-amino)-2-butoxy-7-iodo-5-((2-(trimethylsilyl)ethoxy)me thyl)pyrrolo[3,2-*d*]pyrimidine-6-nitrile (49.70g, crude) as yellow oil, which was used for the next step directly. MS (m/z): 688 [M+H⁺].

### Step F: synthesis of 4-amino-2-butoxy-7-iodo-5-((2-(trimethylsilyl)ethoxy) methyl)pyrrolo[3,2-d]pyrimidine-6-nitrile

4-((Bis-*tert*-butoxycarbonyl)-amino)-2-butoxy-7-iodo-5-((2-(trimethylsilyl)ethoxy)me thyl)pyrrolo[3,2-*d*]pyrimidine-6-nitrile (49.70g) was dissolved in anhydrous dichloromethane (440mL), trifluoroacetic acid (84.15g) was slowly added at 20-25°C. The reaction mixture was stirred for 20 hours at 20-25°C. After the reaction was complete as monitored by TLC, it was concentrated under reduced pressure and the residue was dissolved in dichloromethane (600mL). The solution was basified to pH between 8-9 by the addition of saturated aqueous sodium bicarbonate solution. The organic phase was separated, washed with saturated brine solution (300mL), and dried over anhydrous sodium sulfate and filtered. The filtrate was concentrated under reduced pressure. To the residue was added hexane/ethyl acetate (10/1, 250 mL) and the suspension was stirred vigorously for 1 hour and then filtered. The filter cake was dried under reduced pressure to give 4-amino-2-butoxy-7-iodo-5-((2-(trimethylsilyl)ethoxy)methyl)pyrrolo[3,2-*d*]pyrimidi ne-6-carbonitrile (30.80g, 58.77mmol, yield for 2 sreps: 81.31%) as a yellow solid. **¹H** NMR (400 MHz, CHLOROFORM-*d*): δ 6.08 br. s., 2H), 5.64 (s, 2H), 4.39 (t, *J* = 6.6 Hz, 2H), 3.67-3.76 (m, 2H), 1.73-1.86 (m, 2H), 1.48-1.56 (m, 2H), 0.95-1.04 (m, 5H), 0.00 (m, 9H).

### Step G: synthesis of 4-amino-2-butoxy-7-(5-chloro-1-pentynyl)-5-((2-(trimethylsilyl) ethoxy)methyl)pyrrolo[3,2-d]pyrimidine-6-carbonitrile

A dry three-necked round flask was filled with nitrogen, and then charged with anhydrous acetonitrile (125mL), 4-amino-2-butoxy-7-iodo-5-((2-(trimethylsilyl) ethoxy)methyl)pyrrolo[3,2-*d*]pyrimidine-6-carbonitrile (10g 20.52mmol), triethylamine (6.23g, 61.56mmol), copper(I) iodide (390.74mg, 2.05mmol) and bis(triphenylphosphine)palladium (II) dichloride (1.44g, 2.05mmol). The reaction system was degassed and refilled with nitrogen thrice, and then stirred for 16 hours at room temperature (25 °C). After the reaction was complete as monitored by LC-MS, the mixture was filtered, and the fiter cake was dissolved in dichloromethane (120mL). Then thereto was added activated carbon (5g), and the resulting mixture was stirred at room temperature for 1 hour and filtered. The filter cake was washed with dichloromethane until no desired product could be monitored by TLC. The combined filtrate was washed with diluted aqueous ammonia (200mL × 3). The organic layer was dried over anhydrous sodium sulfate and fitered. The filtrate was concentrated under reduced pressure. To the residue was added hexane/ethyl acetate (20/1, 80mL) and the suspension was stirred vigorously for 1 hour and then filtered. The filter cake was dried under reduced pressure to give 4-amino-2-butoxy-7-(5-chloro-1-pentynyl)-5-((2-(trimethylsilyl)ethoxy)methyl)pyrrol o[3,2-*d*]pyrimidine-6-carbonitrile (8.20g, 14.73mmol, yield: 71.78%) as an off-white solid. **¹H** NMR (400 MHz, CHLOROFORM-*d*): δ 5.59 (s, 2H), 4.38 (t, *J* = 6.6 Hz, 2H), 3.81 (t, *J* = 6.3 Hz, 2H), 3.64-3.72 (m, 2H), 2.75 (t, *J* = 6.8 Hz, 2H), 2.13 (t, *J* = 6.5 Hz, 2H), 1.74-1.84 (m, 2H), 1.51 (d, *J* = 7.5 Hz, 2H), 0.94-1.04 (m, 5H), 0.00 (s, 9H).

### Step H: synthesis of 4-amino-2-butoxy-7-(5-chloro-1-pentynyl)-5-((2-(trimethylsilyl) ethoxy)methyl)pyrrolo[3,2-d]pyrimidine-6-carbonitrile

4-Amino-2-butoxy-7-(5-chloro-1-pentynyl)-5-((2-(trimethylsilyl)ethoxy)methyl)pyrro lo[3,2-*d*]pyrimidine-6-carbonitrile (8.00g, 17.16mmol) was placed in ethanol (400mL), nitrogen was charged for three times, and thereto was added dry 10% Pd/C (6.00g) in one portion. The resulting mixture was then was charged with hydrogen for three times, the reaction mixture was stirred under 50psi hydrogen at 40°C for 16 hours. After the reaction was complete as monitored by TLC, the reaction mixture was cooled to room temperature, and filtered through a celite pad. The filter cake was washed with dichloromethane (200mL×3). The combined organic phase was concentrated under reduced pressure to give 4-amino-2-butoxy-7-(5-chloro-1-pentyl)-5-((2-(trimethylsilyl)ethoxy)methyl)pyrrolo[3,2-*d*]pyrimidine-6-carbonitrile (8.00g, 17.16mmol) as an off-white solid. **¹H** NMR (400MHz, CHLOROFORM-*d*): δ 5.57 (s, 2H), 4.33 (t, *J* = 6.6 Hz, 2H), 3.64-3.71 (m, 2H), 3.54 (t, *J* = 6.7 Hz, 2H), 2.85 (t, *J* = 7.5 Hz, 2H), 1.74-1.90 (m, 6H), 1.46-1.55 (m, 4H), 0.94-1.03 (m, 5H), 0.00 (s, 9H).

### Step I: synthesis of 4-amino-2-butoxy-7-(5-(morpholin-1-yl)pentyl)-5-((2-(trimethylsilyl)ethoxy)methyl)pyrrolo[3,2-d]pyrimidine-6-carbonitrile

4-Amino-2-butoxy-7-(5-chloro-1-pentyl)-5-((2-(trimethylsilyl)ethoxy)methyl)pyrrolo[ 3,2-*d*]pyrimidine-6-carbonitrile (100mg, 214.55µmol) was dissolved in acetonitrile (5mL), morpholine (56.07mg, 643.65µmol), triethylamine (21.71mg, 214.55µmol), and sodium iodide (3.22mg, 21.46µmol) were added sequentially. The reaction mixture was heated to 75-85°C and then stirred for 14 hours. The reaction mixture was cooled to room temperature, diluted with water (20mL), and extracted with ethyl acetate (20mL×2). The combined organic layer was washed with saturated brine (30mL), dried over anhydrous sodium sulfate and fitered. The filtrate was concentrated under reduced pressure to give 4-amino-2-butoxy-7-(5-(morpholin-1-yl)pentyl)-5-((2-(trimethylsilyl)ethoxy)methyl)p yrrolo[3,2-*d*]pyrimidine-6-carbonitrile (102mg, crude), which was used for the next step directly. MS (ESI) m/z: 517 [M+H⁺].

### Step J: synthesis of 4-amino-2-butoxy-7-(5-(1-morpholinyl)pentyl)-5H pyrrolo[3,2-d]pyrimidine-6-carbonitrile

4-Amino-2-butoxy-7-(5-(1-morpholinyl)pentyl)-5-((2-(trimethylsilyl)ethoxy)methyl)p yrrolo[3,2-*d*]pyrimidine-6-carbonitrile (102mg, crude) was dissolved in trifluoroacetic acid (2mL). The reaction mixture was stirred at 15-20°C for 14 hours. After the reaction was complete as monitored by LC-MS, the reaction mixture was concentrated under reduced pressure. The residue was purified by prep-HPLC (FA condition), and then lyophilized to give 4-amino-2-butoxy-7-(5-(1-morpholino)pentyl)-5*H*-pyrrolo[3,2-*d*]pyrimidine-6-carbon itrile formate (47.89mg). **¹H** NMR (400 MHz METHANOL-d₄): δ 8.32 (br. s., 2H), 4.33 (t, *J* = 6.6 Hz, 2H), 3.88 (d, *J* = 4.3 Hz, 4H), 3.21 (br. s., 4H), 3.08-3.02 (m, 2H), 2.82 (t, *J* = 7.3 Hz, 2H), 1.83-1.72 (m, 6H), 1.55-1.39 (m, 4H), 0.99 (t, *J* = 7.4 Hz, 3H). MS (ESI) m/z: 387 [M+H⁺].

### Example 5

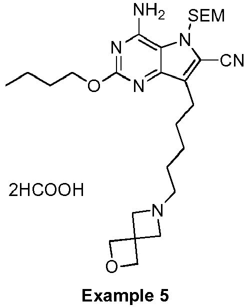

### 7-(5-(2-oxa-6-azaspiro[3.3]heptan-6-yl)pentyl)-4-amino-2-butoxy-5H-pyrrolo[3,2-d]p yrimidine-6-carbonitrile formate

### Step A: synthesis of 4-amino-2-butoxy-7-(5-(2-oxa-6-azaspiro[3.3]heptan-6-yl)pentyl) -5-((2-(trimethylsilyl)ethoxy)methyl)pyrrolo[3,2-d]pyrimidine-6-carbonitrile

4-Amino-2-butoxy-7-(5-(2-oxa-6-azaspiro[3.3]heptan-6-yl)pentyl)-5-((2-(trimethylsil yl)ethoxy)methyl)pyrrolo[3,2-*d*]pyrimidine-6-carbonitrile was synthesized according to step I in Example 4, except for replacing the morphine with 2-oxa-6-azaspiro[3.3]heptane. MS (ESI) m/z: 529 [M+H⁺].

### Step B: synthesis of 4-amino-2-butoxy-7-(5-(2-oxa-6-azaspiro[3.3] heptan-6-yl)pentyl)-5H-pyrrolo[3,2-d]pyrimidine-6-carbonitrile formate

To a solution of 4-amino-2-butoxy-7-(5-(2-oxa-6-azaspiro[3.3]heptan-6-yl) pentyl)-5*H*-((2-(trimethylsilyl)ethoxy)methyl)pyrrolo[3,2-*d*]pyrimidine-6-carbonitrile (49.00mg, 92.67mmol) in tetrahydrofuran (2.00mL), was added a solution of tetrabutylammonium fluoride in THF (1M, 185.34 µL). The mixture was heated to 60 °C and stirred for 14 hrs. The mixture was cooled to room temperature and poured into water (20mL), extracted with dichloromethane/methanol (10/1, 20mL × 2). The combined organic layer was washed with saturated brine (30mL), dried over anhydrous sodium sulfate, filtered and concentrated under reduced pressure. The residue was dissolved in methanol (4mL) and potassium carbonate (70mg) was added to the mixture. The mixture was stirred at 40 °C for further 1 hr. After cooling, the mixture was filtered and the filtrate was concentrated under reduced pressure. The residue was purified by pre-HPLC (FA condition). After lyophilization, 4-amino-2-butoxy-7-(5-(2-oxa-6-azaspiro[3.3]heptan-6-yl)pentyl)-5*H*-pyrrolo[3,2-*d*]p yrimidine-6-carbonitrile formate was given as white solid. (15.59mg, yield 37.84%). **¹H** NMR (400 MHz, METHANOL-d₄): δ 8.33 (br. s., 2H), 4.78 (s, 4H), 4.46-4.17 (m, 6H), 3.16-3.05 (m, 2H), 2.81 (t, *J* = 7.3 Hz, 2H), 1.82-1.69 (m, 4H), 1.63-1.36 (m, 6H), 0.99 (t, *J* = 7.4 Hz, 3H). MS (ESI) m/z: 399 [M+H⁺].

### Example 6

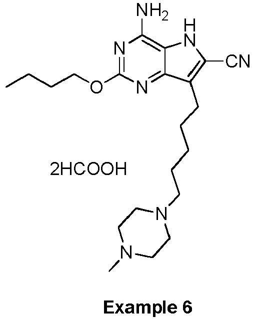

### 4-amino-2-butoxy-7-(5-(4-methylpiperazin-1-yl)pentyl)-5H-pyrrolo[3,2-d]pyrimidine -6-carbonitrile formate

4-Amino-2-butoxy-7-(5-(4-methylpiperazin-1-yl)pentyl)-5*H*-pyrrolo[3,2-*d*]pyrimidin e-6-carbonitrile formate was synthesized according to steps I, J in Example 4, except for replacing the morphine with 4-methylpiperazine. **¹H** NMR (METHANOL-*d*₄, 400 MHz): δ 8.23 (d, *J* = 14.4 Hz, 2H), 4.41-4.26 (m, 2H), 3.29-2.83 (m, 8H), 2.83-2.70 (m, 4H), 2.62 (s, 3H), 1.82-1.70 (m, 4H), 1.70-1.60 (m, 2H), 1.56-1.46 (m, 2H), 1.45-1.36 (m, 2H), 0.99 (t, *J* = 7.4 Hz, 3H). MS (ESI) m/z: 400 [M+H⁺].

### Example 7

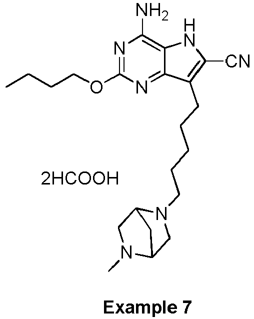

### 4-amino-2-butoxy-7-(5-(5-methyl-2,5-diazabicyclo[2.2.1]heptan-2-yl)pentyl)-5H-pyrr olo[3,2-d]pyrimidine-6-carbonitrile formate

4-Amino-2-butoxy-7-(5-(5-methyl-2,5-diazabicyclo[2.2.1]heptan-2-yl)pentyl)-5*H-*pyr rolo[3,2-*d*]pyrimidine-6-carbonitrile formate was synthesized according to steps I, J in Example 4, except for replacing the morphine with 5-methyl-2,5-diazabicyclo[2.2.1] heptane. **¹H** NMR (400 MHz, METHANOL-*d*₄): δ 8.26 (br. s., 2H), 4.34 (t, *J* = 6.5 Hz, 2H), 3.88 (d, *J* = 18.3 Hz, 2H), 3.22-2.77 (m, 7H), 2.68 (s, 3H), 2.17-2.02 (m, 2H), 1.82-1.71 (m, 4H), 1.70-1.14 (m, 7H), 0.99 (t, *J* = 7.4 Hz, 3H). MS (ESI) m/z: 412 [M+H⁺] .

### Example 8

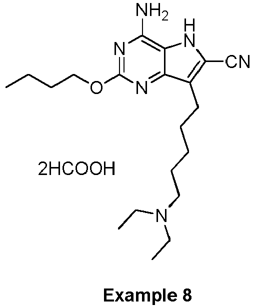

### 4-amino-2-butoxy-7-(5-(diethylamino)pentyl)-5H-pyrrolo[3,2-d]pyrimidine-6-carboni trile formate

4-Amino-2-butoxy-7-(5-(diethylamino)pentyl)-5*H*-pyrrolo[3,2-*d*]pyrimidine-6-carbon itrile formate was synthesized according to steps I, J in Example 4, except for replacing the morphine with diethylamine hydrochloride and increasing the equivalent of triethylamine to 3. **¹H** NMR (400 MHz, METHANOL-*d*₄): δ 8.37 (br. s., 2H), 4.32 (t, *J* = 6.6 Hz, 2H), 3.20 (q, *J* = 7.3 Hz, 4H), 3.14-3.05 (m, 2H), 2.84 (t, *J* = 7.3 Hz, 2H), 1.86-1.67 (m, 6H), 1.56-1.39 (m, 4H), 1.29 (t, *J* = 7.3 Hz, 6H), 0.99 (t, *J* = 7.3 Hz, 3H). MS (ESI) m/z: 373 [M+H⁺].

### Example 9

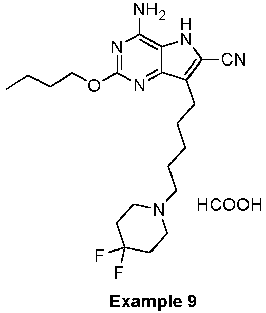

### 4-amino-2-butoxy-7-(5-(4,4_{,}-difluoro-1-pipcridinyl)pentyl)-5H-pyrrolo[3,2-d]pyrimid ine-6-carbonitrile formate

4-Amino-2-butoxy-7-(5-(4,4'-difluoro-1-piperidinyl)pentyl)-5H-pyrrolo[3,2-*d*]pyrimi dine-6-carbonitrile formate was synthesized according to steps I, J in Example 4, except for replacing the morphine with 4,4'-difluoropiperidine. **¹H** NMR (400 MHz, METHANOL *-d*₄): δ 8.21 (s, 1H), 4.37 (t, *J* = 6.5 Hz, 2H), 3.37 (t, *J* = 5.5 Hz, 4H), 3.15-3.07 (m, 2H), 2.82 (t, *J* = 7.4 Hz, 2H), 2.31 (tt, *J* = 6.1, 12.7 Hz, 4H), 1.83-1.72 (m, 6H), 1.55-1.40 (m, 4H), 0.99 (t, *J* = 7.4 Hz, 3H). MS (ESI) m/z: 421 [M+H⁺].

### Example 10

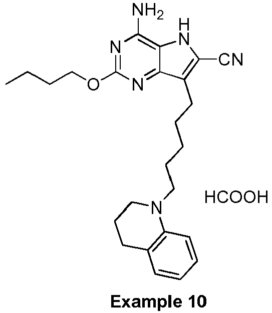

### 4-amino-2-butoxy-7-(5-(1-tetrahydroquinolinyl)pentyl)-5H-pyrrolo[3,2-d]pyrimidine-6-carbonitrile formate

4-Amino-2-butoxy-7-(5-(1-tetrahydroquinolinyl)pentyl)-5*H*-pyrrolo[3,2-*d*]pyrimidine -6-carbonitrile formate was synthesized according to steps I, J in Example 4, except for replacing the morphine with tetrahydroquinoline. **¹H** NMR (400 MHz, METHANOL-*d*₄): δ 8.45 (br. s., 1H), 6.89-6.97 (m, 1H), 6.84 (d, *J* = 7.4 Hz, 1H), 6.53 (d, *J* = 8.3 Hz, 1H), 6.46 (t, *J* = 7.3 Hz, 1H), 4.33 (t, *J* = 6.6 Hz, 2H), 3.18-3.29 (m, 4H), 2.84 (t, *J* = 7.3 Hz, 2H), 2.69 (t, *J* = 6.3 Hz, 2H), 1.84-1.93 (m, 2H), 1.72-1.83 (m, 4H), 1.64 (t, *J* = 7.5 Hz, 2H), 1.47-1.54 (m, 2H), 1.40 (t, *J =* 7.5 Hz, 2H), 0.99 (t, *J* = 7.4 Hz, 3H). MS (ESI) m/z: 433 [M+H⁺].

### Example 11

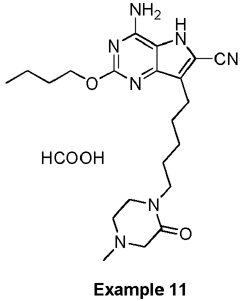

### 4-amino-2-butoxy-7-(5-(4-methyl-2-oxopiperazin-1-yl)pentyl)-5H-pyrrolo[3,2-d] pyrimidine-6-carbonitrile formate

### Route: Preparation of Example 11

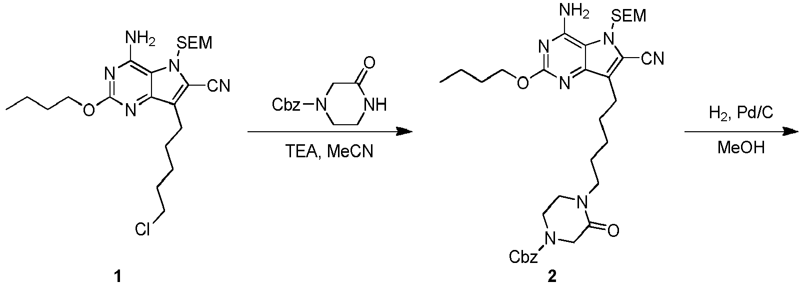

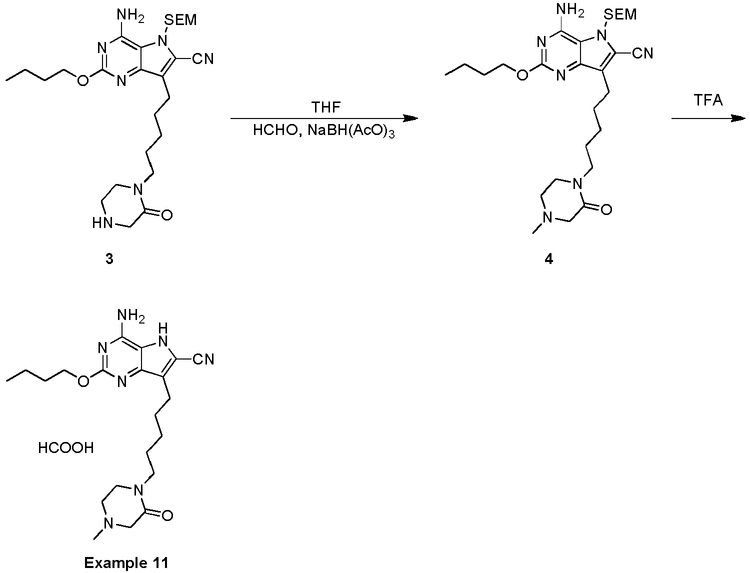

### Step A: synthesis of 4-amino-2-butoxy-7-(5-(4-benzoxycarbonyl-2-oxo-piperazidin-1-yl)pentyl)-5-((2-(trimethylsilyl)ethoxy)methyl)pyrrolo[3,2-d]pyrimidin-6-nitrile

4-Benzyloxycarbonyl-2-piperazinone (190.00mg, 811.97µmol) was dissolved in anhydrous tetrahydrofuran (5mL), sodium hydride (60%, 35.73mg, 893.16µmol) was added in one portion at 0°C under nitrogen atmosphere. The reaction mixture was stirred at 0 °C for 1 hr. Then 4-amino-2-butoxy-7-[5-(1-chloro)pentyl]-5-((2-(trimethylsilyl)ethoxy)methyl)pyrrolo [3,2-*d*]pyrimidine-6-carbonitrile (250mg, 537.63µmol) was added to the mixture. The resulting reaction mixture was warmed to room temperature and stirred for further 24 hrs. After that, the mixture was quenched by saturated aqueous ammonium chloride solution (5mL), diluted with water (20mL) and extracted with ethyl acetate (20mL × 2). The combined organic layer was washed with saturated brine, dried over anhydrous sodium sulfate, filtered and the filtrate was concentrated under reduced pressure. The residue was purified by pre-TLC (dichloromethane/methanol=15:1) to give 4-amino-2-butoxy-7-(5-(4-benzoxycarbonyl-2-oxo-piperazidin-1-yl)pentyl)-5-((2-(trimethylsilyl)ethoxy)methyl) pyrrolo[3,2-*d*]pyrimidin-6-nitrile (220mg, yield 61.62%). MS (ESI) m/z: 663 [M+H⁺] .

### Step B: synthesis of 4-amino-2-butoxy-7-(5-(2-oxo-piperazidin-1-yl)pentyl)-5-((2-(trimethylsilyl)ethoxy) methyl)pyrrolo[3,2-d]pyrimidin-6-nitrile

4-Amino-2-butoxy-7-(5-(4-benzoxycarbonyl-2-oxo-piperazidin-1-yl)pentyl)-5-((2-(tri methylsilyl)ethoxy)methyl)pyrrolo[3,2-*d*]pyrimidin-6-nitrile (200.00mg, 303.10µmol) was dissolved in a mixed solvent of ethanol (10mL) and methanol (5mL). Pd/C (100mg) was added to the mixture under nitrogen atmosphere. The mixture was stirred at 10 to 15°C under hydrogen (50psi) for 36 hrs. The mixture was filtered through celite pad and the filtrate was concentrated under reduced pressure to give 4-amino-2-butoxy-7-(5-(2-oxopiperazin-1-yl)pentyl)-5-((2-(trimethylsilyl)ethoxy)met hyl)pyrrolo[3,2-*d*]pyrimidine-6-carbonitrile (130mg, crude) as yellow oil. The residue was used for the next step directly. MS (ESI) m/z: 530 [M+H⁺].

### Step C: synthesis of 4-amino-2-butoxy-7-(5-(4-methoxy-2-oxopiperazin-1-yl) pentyl)-5-((2-(trimethylsilyl)ethoxy)methyl)pyrrolo[3,2-d]pyrimidine-6-carbonitrile

4-Amino-2-butoxy-7-(5-(2-oxopiperazin-1-yl)pentyl)-5-((2-(trimethylsilyl)ethoxy)me thyl)pyrrolo[3,2-*d*]pyrimidine-6-carbonitrile (130.00mg, 245.40µmol) was dissolved in tetrahydrofuran (3mL), formaldehyde (22.11mg, 736.20µmol), sodium triacetoxyborohydride (130.03mg, 613.50µmol) and a catalytic amount of acetic acid were added sequentially. The mixture was stirred at 15 to 20 °C for 3 hrs, and then, poured into saturated aqueous sodium bicarbonate solution (10mL) and extracted with ethyl acetate (10mL). The organic layer was dried over anhydrous sodium sulfate, filtered and the filtrate was concentrated under reduced pressure to give 4-amino-2-butoxy-7-(5-(4-methyl-2-oxopiperazin-1-yl)pentyl)-5-((2-(trimethylsilyl)et hoxy)methyl)pyrrolo[3,2-*d*]pyrimidine-6-carbonitrile (150mg, crude) which was used for the next step directly. MS (ESI) m/z: 544 [M+H⁺].

### Step D: synthesis of 4-amino-2-butoxy-7-(5-(4-methyl-2-oxopiperazin-1-yl) pentyl)-5H-pyrrolo[3,2-d]pyrimidine-6-carbonitrile formate

4-Amino-2-butoxy-7-(5-(4-methyl-2-oxopiperazin-1-yl)pentyl)-5-((2-(trimethylsilyl)e thoxy)methyl)pyrrolo[3,2-*d*]pyrimidine-6-carbonitrile (150mg, 275.85µmol) was dissolved in trifluoroacetic acid (1.5mL). The mixture was stirred at 15 to 20°C for 12 hrs. The mixture was concentrated under reduced pressure to remove trifluoroacetic acid and neutralized with saturated aqueous sodium bicarbonate solution (30mL). The mixture was extracted with ethyl acetate (30mL × 1). The organic layer was dried over anhydrous sodium sulfate, filtered and the filtrate was concentrated under reduced pressure to give a brown solid. This solid was dissolved in methanol (10mL) and potassium carbonate (120mg) was added. The mixture was stirred at 40 to 50°C for further 1 hr and filtered. The filtrate was concentrated under reduced pressure and the residue was purified by pre-HPLC (FA condition) to obtain 4-amino-2-butoxy-7-(5-(4-methyl-2-oxopiperazin-1-yl)pentyl)-5*H-*pyrrolo [3,2-*d*]pyrimidine-6-carbonitrile formate (23.50mg, 46.74µmol, yield: 16.94%). **¹H** NMR (400 MHz, METHANOL-*d*₄): δ 8.21 (br. s., 1H), 4.39 (t, *J* = 6.6 Hz, 2H), 3.45-3.36 (m, 4H), 3.14 (s, 2H), 2.87-2.72 (m, 4H), 2.40 (s, 3H), 1.83-1.72 (m, 4H), 1.64-1.51 (m, 4H), 1.38-1.32 (m, 2H), 1.01 (t, *J* = 7.4 Hz, 3H). MS (ESI) m/z: 414 [M+H⁺] .

### Example 12

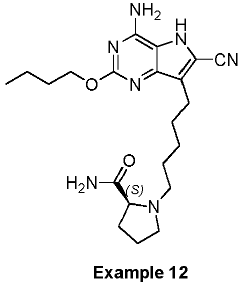

### 4-amino-2-butoxy-7-(5-(2-(S)-formamido-pyrrolidin-1-yl)pentyl)-5H-pyrrolo[3,2-d]p yrimidin-6-nitrile

### Step A: synthesis of 4-amino-2-butoxy-7-(5-(2-(S)-formamido-pyrrolidin-1-yl)pentyl) -5-((2-(trimethylsilyl)ethoxy)methyl)pyrrolo[3,2-d]pyrimidin-6-nitrile

4-Amino-2-butoxy-7-(5-chloro-1-pentyl)-5-((2-(trimethylsilyl)ethoxy)methyl)pyrrolo[ 3,2-*d*]pyrimidine-6-carbonitrile (100mg, 214.55µmol) was added to acetonitrile (3mL), 2-(*S*)-methyl prolinate hydrochloride (71.07mg, 429.10µmol), potassium carbonate (88.96mg, 644.64µmol), and sodium iodide (3.22mg, 21.46µmol) were added sequentially. The reaction mixture was stirred at 75 to 85°C for 14 hr. LC-MS showed that the chlororeagent was not consumed completely. Additionaly potassium carbonate (88.96mg, 644.64µmol) was added. The mixture was stirred at 75 to 85°C for further 14 hr. LC-MS showed that the conversion of chlororeagent reached 72%. After the mixture was cooled to room temperature, 20mL water was added. The resulting mixture was extracted with ethyl acetate (20mL × 2). The combined organic layer was washed by saturated brine (30mL), dried over anhydrous sodium sulfate, filtered and the filtrate was concentrated under reduced pressure to give 4-amino-2-butoxy-7-(5-(2-(S)-formamido-pyrrolidin-1-yl)pentyl)-5-((2-(trimethylsilyl)ethoxy)methyl)pyrrolo[3,2-*d*]pyrimidin-6-nitrile (115.00mg, crude) which was used for next step directly. MS (ESI) m/z: 559 [M+H⁺]

### Step B: synthesis of 4-amino-2-butoxy-7-(5-(2-(S)-methoxycarbonyl-pyrrolidin-1-yl) pentyl)-5H-pyrrolo[3,2-d]pyrimidin-6-nitrile

4-Amino-2-butoxy-7-(5-(2-(S)-formamido-pyrrolidin-1-yl)pentyl)-5-((2-(trimethylsily l)ethoxy)methyl)pyrrolo[3,2-*d*]pyrimidin-6-nitrile (115.00mg, crude) was dissolved in trifluoroacetic acid (2mL). The solution was stirred at 15 to 20 °C for 14 hr. LC-MS showed that the reaction was complete. The solution was concentrated under reduced pressure to give a residue. This residue was diluted with 30mL saturated aqueous sodium bicarbonate solution and extracted with dichloromethane (20mL × 2). The combined organic layer was dried over anhydrous sodium sulfate, filtered and the filtrate was concentrated under reduced pressure, dried to give 4-amino-2-butoxy-7-(5-(2-(S)-methoxycarbonyl-pyrrolidin-1-yl)pentyl)-5*H*-pyrrolo[3 ,2-*d*]pyrimidin-6-nitrile (88.00 mg, crude) which was used for next step directly. MS (ESI) m/z: 429 [M+H⁺].

### Step C: synthesis of 4-amino-2-butoxy-7-(5-(2-(S)-formamido-pyrrolidin-1-yl) pentyl)-5H-pyrrolo[3,2-d]pyrimidin-6-nitrile

4-Amino-2-butoxy-7-(5-(2-(S)-formamido-pyrrolidin-1-yl)pentyl)-5*H*-pyrrolo[3,2-*d*]p yrimidin-6-nitrile (88.00mg, crude) was dissolved in methanol (3mL) at room temperature, lithium hydroxide monohydrate (17.25mg, 410.70µmol) was added in one portion. The mixture was stirred at room temperature for 14 hr. LC-MS showed that the reation was complete. The reaction solution was poured into saturated aqueous ammonium chloride solution (20mL) and extracted with dichloromethane/methanol (10/1, v/v, 20mL × 3). The combined organic layer was dried over anhydrous sodium sulfate, filtered and the filtrate was concentrated under reduced pressure. The residue was purified by prep-HPLC (FA condition) to give 4-amino-2-butoxy-7-(5-(2-(S)-carboxyl-pyrrolidin-1-yl)pentyl)-5H-pyrrolo[3,2-*d*]pyri midin-6-nitrile (22.07mg, purity 97%) as a white solid. **¹H** NMR (400 MHz, METHANOL-*d*₄): δ 8.22 (s, 1H), 4.34 (t, *J* = 6.6 Hz, 2H), 3.85 (dd, *J* = 6.2, 9.2 Hz, 1H), 3.72 (ddd, *J* = 3.8, 7.1, 11.1 Hz, 1H), 3.28-3.19 (m, 1H), 3.15-3.04 (m, 2H), 2.80 (t, *J* = 7.4 Hz, 2H), 2.42 (qd, *J* = 8.7, 13.5 Hz, 1H), 2.17-2.04 (m, 2H), 2.00-1.90 (m, 1H), 1.83-1.69 (m, 6H), 1.55-1.39 (m, 4H), 0.99 (t, *J* = 7.4 Hz, 3H).

### Step D: synthesis of 4-amino-2-butoxy-7-(5-(2-(S)-formamido-pyrrolidin-1-yl) pentyl)-5H-pyrrolo[3,2-d]pyrimidin-6-nitrile

4-Amino-2-butoxy-7-(5-(2-(S)-carboxyl-pyrrolidin-1-yl)pentyl)-5H-pyrrolo[3,2-*d*]pyr imidin-6-nitrile (50.00mg, 120.63µmol) was dissolved in *N,N-*dimethylformamide (3mL) at room temperature, ammonium carbonate (115.91mg, 1.21mmol), triethylamine (24.41mg, 241.25µmol) and HATU (68.76mg, 180.95µmol) were added sequentially and then sealed. The mixture was stirred at 40 to 50°C for 16 hr. LC-MS showed that the reaction was complete. The mixture was diluted with water (20mL) and extracted with ethyl acetate (20mL × 2). The combined organic layer was dried over anhydrous sodium sulfate, filtered and the filtrate was concentrated under reduced pressure. The residue was purified by prep-HPLC (FA condition), lyophilized to give 4-amino-2-butoxy-7-(5-(2-(S)-formamido-pyrrolidin-1-yl) pentyl)-5H-pyrrolo[3,2-*d*]pyrimidin-6-nitrile (20mg) as a white solid. **¹H** NMR (400 MHz, METHANOL-*d*₄): δ 4.50 (t, *J* = 6.5 Hz, 2H), 4.18 (dd, *J* = 7.0, 9.2 Hz, 1H), 3.77 (dd, *J* = 4.0, 7.2, 11.3 Hz, 1H), 3.29-3.12 (m, 3H), 2.83 (t, *J* = 7.4 Hz, 2H), 2.66-2.49 (m, 1H), 2.26-1.96 (m, 3H), 1.87-1.67 (m, 6H), 1.59-1.40 (m, 4H), 1.02 (t, *J* = 7.4 Hz, 3H). MS (ESI) m/z: 414 [M+H⁺].

### Example 13

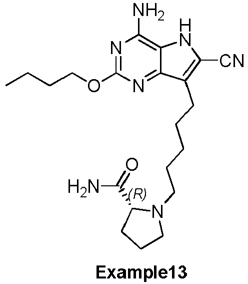

### 4-amino-2-butoxy-7-(5-(2-(R)-formamido-pyrrolidin-1-yl)pentyl)-5H-pyrrolo[3,2-d]p yrimidin-6-nitrile

4-amino-2-butoxy-7-(5-(2-(S)-formamido-pyrrolidin-1-yl)pentyl)-5H-pyrrolo[3,2-*d*]p yrimidin-6-nitrile was synthesized according to the steps A-D in example 12, except for replacing 2-(*S*)-methyl prolinate hydrochloride with 2-(*R*)-methyl prolinate hydrochloride. **¹H** NMR (400 MHz, METHANOL-*d*₄)*:* δ 4.53 (t, *J* = 6.53 Hz, 2H), 4.17 (dd, *J* = 7.03, 9.03 Hz, 1H), 3.72-3.82 (m, 1H), 3.12-3.28 (m, 3H), 2.83 (t, *J* = 7.40 Hz, 2H), 2.51-2.63 (m, 1H), 1.97-2.26 (m, 3H), 1.67-1.88 (m, 6H), 1.40-1.59 (m, 4H), 1.02 (t, *J* = 7.40 Hz, 3H). MS (ESI) m/z: 414 [M+H⁺].

### Example 14

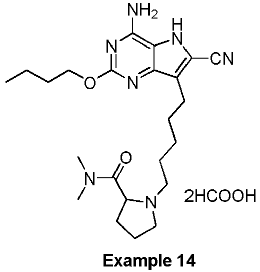

### 4-amino-2-butoxy-7-(5-(2-(N,N'-dimethylformamido)-pyrrolidin-1-yl)pentyl)-5H-pyr rolo[3,2-d]pyrimidin-6-nitrile formate

### Step A: synthesis of 4-amino-2-butoxy-7-(5-(2-carboxyl-pyrrolidin-1-yl)pentyl)-5H-pyrrolo[3,2-d]pyrimidin-6-nitrile

4-Amino-2-butoxy-7-(5-(2-carboxyl-pyrrolidin-1-yl)pentyl)-5H-pyrrolo[3,2-*d*]pyrimi din-6-nitrile was synthesized according to the steps A-C in example 12, except for replacing 2-(*S*)-methyl prolinate hydrochloride with 2-(R,S)-methyl prolinate.

### Step B: synthesis of 4-amino-2-butoxy-7-(5-(2-(N,N'-dimethylformamido)-pyrrolidin-1-yl)pentyl)-5H-pyrrolo[3,2-d]pyrimidin-6-nitrile formate

4-Amino-2-butoxy-7-(5-(2-(N,N'-dimethylformamido)-pyrrolidin-1-yl)pentyl)-5H-py rrolo[3,2-*d*]pyrimidin-6-nitrile formate was prepared according to step D in example 12, except for replacing ammonium carbonate with dimethylamine hydrochloride. **¹H** NMR (400 MHz, METHANOL-*d*₄): δ 8.41 (br. s., 2H), 4.59 (dd, *J* = 6.6, 9.5 Hz, 1H), 4.34 (t, *J* = 6.5 Hz, 2H), 3.80-3.78 (m, 1H), 3.25-3.10 (m, 3H), 3.07 (s, 3H), 3.03 (s, 3H), 2.83 (t, *J* = 7.3 Hz, 2H), 2.69-2.55 (m, 1H), 2.26-2.14 (m, 1H), 2.04-1.93 (m, 2H), 1.86-1.67 (m, 6H), 1.58-1.38 (m, 4H), 1.01 (t, *J* = 7.4 Hz, 3H). MS (ESI) m/z: 442 [M+H⁺] .

### Example 15

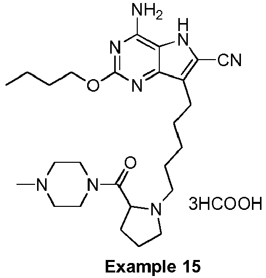

### 4-amino-2-butoxy-7-(5-((2-(4-methylpiperazine-1-carbonyl)pyrrolidin-1-yl)pentyl)-5 H-pyrrolo[3,2-d]pyrimidine-6-carbonitrile formate

### Step A: synthesis of 4-amino-2-butoxy-7-(5-(2-carboxyl-pyrrolidin-1-yl)pentyl)-5H-pyrrolo[3,2-d]pyrimidine-6-carbonitrile

4-Amino-2-butoxy-7-(5-(2-carboxyl-pyrrolidin-1-yl)pentyl)-5*H*-pyrrolo[3,2-*d*]pyrimi dine-6-carbonitrile was synthesized according to the steps A-C in example 12, except for replacing 2-(*S*)-methyl prolinate hydrochloride with 2-(R,S)-methyl prolinate.

### Step B: synthesis of 4-amino-2-butoxy-7-(5-((2-(4-methylpiperazidin-1-carbonyl)) -pyrrolidin-1-yl)pentyl)-5H-pyrrolo[3,2-d]pyrimidine-6-carbonitrile formate

4-Amino-2-butoxy-7-(5-((2-(4-methylpiperazidin-1-carbonyl))-pyrrolidin-1-yl)pentyl) -5*H*-pyrrolo[3,2-*d*]pyrimidine-6-carbonitrile formate was prepared according to the step D in example 12, except for replacing ammonium carbonate with 4-methyl piperazine. **¹H** NMR (400MHz, METHANOL-*d*₄) δ 8.29 (br. s., 3H), 4.61 (dd, *J* = 6.6, 9.0 Hz, 1H), 4.35 (t, *J* = 6.6 Hz, 2H), 3.87-3.49 (m, 5H), 3.27-3.08 (m, 3H), 2.84 (t, *J* = 7.3 Hz, 2H), 2.74-2.54 (m, 5H), 2.46 (s, 3H), 2.28-2.15 (m, 1H), 2.10-1.95 (m, 2H), 1.86-1.68 (m, 6H), 1.59-1.40 (m, 4H), 1.01 (t, *J* = 7.4 Hz, 3H). MS (ESI) m/z: 497 [M+H⁺].

### Example 16

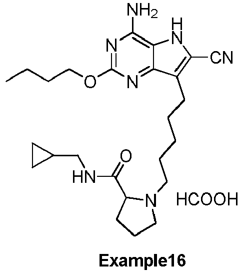

### 4-amino-2-butoxy-7-(5-((2-cyclopropylethylaminocarbonyl)-pyrrolidin-1-yl)pentyl)-5 H-pyrrolo[3,2-d]pyrimidine-6-carbonitrile formate

### Step A: synthesis of 4-amino-2-butoxy-7-(5-(2-carboxyl-pyrrolidin-1-yl)pentyl)-5H pyrrolo[3,2-d]pyrimidine-6-carbonitrile

4-Amino-2-butoxy-7-(5-(2-carboxyl-pyrrolidin-1-yl)pentyl)-5*H*-pyrrolo[3,2-*d*]pyrimi dine-6-carbonitrile was synthesized according to the steps A-C in example 12, except for replacing 2-(*S*)-methyl prolinate with 2-(R,S)-methyl prolinate.

### Step B: synthesis of 4-amino-2-butoxy-7-(5-((2-cyclopropylethylaminocarbonyl)-pyrrolidin-1-yl)pentyl)-5H-pyrrolo[3,2-d]pyrimidine-6-carbonitrile formate

4-Amino-2-butoxy-7-(5-((2-cyclopropylethylaminocarbonyl)-pyrrolidin-1-yl)pentyl)-5*H*-pyrrolo[3,2-*d*]pyrimidine-6-carbonitrile formate was prepared according to the step D in example 12, except for replacing ammonium carbonate with *N*-methyl cyclopropanamine. **¹H** NMR (400 MHz, METHANOL-*d*₄): δ 8.66 (br. s., 1H), 4.59 (t, *J* = 6.5 Hz, 2H), 4.18 (br. s., 1H), 3.79 (br. s., 1H), 3.28-3.12 (m, 5H), 2.85-2.72 (m, 2H), 2.63-2.53 (m, 1H), 2.24- 2.00 (m, 3H), 1.89-1.68 (m, 6H), 1.59-1.43 (m, 4H), 1.06-0.94 (m, 4H), 0.57-0.51 (m, 2H), 0.28-0.22 (m, 2H). MS (ESI) m/z: 468 [M+H⁺] .

### Example 17

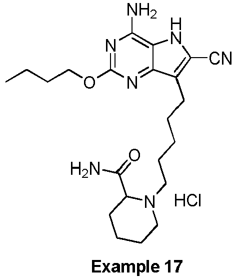

### 4-amino-2-butoxy-7-(5-(2-formamido-piperidin-1-yl)pentyl)-5H-pyrrolo[3,2-d]pyrimi dine-6-carbonitrile hydrochloride

4-Amino-2-butoxy-7-(5-(2-formamido-piperidin-1-yl)pentyl)-5*H-*pyrrolo[3,2-*d*]pyrim idine-6-carbonitrile hydrochloride was synthesized according to the steps A-D in example 12, except for replacing 2-(*S*)-methyl prolinate hydrochloride with 2-(R,S)-methyl pipecolinate hydrochloride. Product was purified by prep-HPLC (HCl condition) and lyophilized. **¹H** NMR (400 MHz, METHANOL-*d*₄): δ 4.59 (t, *J* = 6.5 Hz, 2H), 3.91 (dd, *J* = 2.9, 12.1 Hz, 1H), 3.72-3.65 (m, 1H), 3.18-3.01 (m, 3H), 2.87-2.80 (m, 3H), 2.21 (d, *J* = 14.6 Hz, 1H), 1.95-1.45 (m, 14H), 1.03 (t, *J* = 7.4 Hz, 3H). MS (ESI) m/z: 428 [M+H⁺].

### Example 18

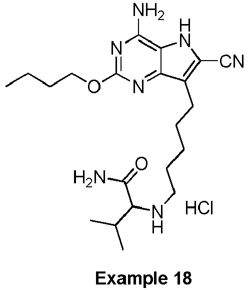

### 4-amino-2-butoxy-7-(5-((1-formamido-1,-isopropyl)amino)pentyl)-5H-pyrrolo[3,2-d] pyrimidine-6-carbonitrile hydrochloride

4-Amino-2-butoxy-7-(5-((1-formamido-1'-isopropyl)amino)pentyl)-5*H*-pyrrolo[3,2-*d* ]pyrimidine-6-carbonitrile hydrochloride was synthesized according to steps A-D in example 12, except for replacing 2-(*S*)-methyl prolinate hydrochloride with methyl 2-amino-3-methylbutanoate. **¹H** NMR (400 MHz, METHANOL-*d*₄): δ 4.56 (t, *J* = 6.6 Hz, 2H), 3.74 (d, *J* = 5.0 Hz, 1H), 3.05-2.92 (m, 2H), 2.86-2.83 (m, 2H), 2.31-2.22 (m, 1H), 1.88-1.69 (m, 6H), 1.59-1.43 (m, 4H), 1.14 (d, *J* = 6.9 Hz, 3H), 1.09 (d, *J* = 6.9 Hz, 3H), 1.03 (t, *J* = 7.4 Hz, 3H). MS (ESI) m/z: 416 [M+H⁺].

### Example 19

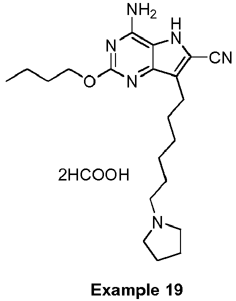

### 4-amino-2-butoxy-7-(6-(tetrahydropyrrol-1-yl)hexyl)-5H-pyrrolo[3,2-d]pyrimidine-6-carbonitrile formate

### Step A: synthesis of 4-amino-2-butoxy-7-(6-chloro-1-hexyl)-5-((2-(trimethylsilyl) ethoxy)methyl)-pyrrolo[3,2-d]pyrimidine-6-carbonitrile

4-Amino-2-butoxy-7-(6-chloro-1-hexyl)-5-((2-(trimethylsilyl)ethoxy)methyl)-pyrrolo [3,2-*d*]pyrimidine-6-carbonitrile was prepared according to steps G and H in example 4, except for replacing 5-chloro-1-pentyne with 6-chloro-1-hexyne.

### Step B: synthesis of 4-amino-2-butoxy-7-(6-(tetrahydropyrrol-1-yl)hexyl)-5H-pyrrolo[3,2-d]pyrimidine-6-carbonitrile formate

4-Amino-2-butoxy-7-(6-(tetrahydropyrrol-1-yl)hexyl)-5*H*-pyrrolo[3,2-*d*]pyrimidine-6 -carbonitrile formate was synthesized according to steps I-J in example 4, except for replacing 4-amino-2-butoxy-7-(5-chloro-1-pentyl)-5-((2-(trimethylsilyl)ethoxy) methyl)pyrrolo[3,2-*d*]pyrimidine-6-carbonitrile with 4-amino-2-butoxy-7-(6-chloro-1-hexyl)-5-((2-(trimethylsilyl)ethoxy)methyl)pyrrolo[3 ,2-*d*]pyrimidine-6-carbonitrile and meanwhile replacing morpholine with tetrahydropyrrole. **¹H** NMR (400 MHz, METHANOL-*d*₄): δ 8.40 (s, 2H), 4.34 (t, *J* = 6.6 Hz, 2H), 3.33 (td, *J* = 1.6, 3.3 Hz, 4H), 3.21-3.11 (m, 2H), 2.82 (t, *J* = 7.3 Hz, 2H), 2.08 (br. s., 4H), 1.86-1.67 (m, 6H), 1.59-1.39 (m, 6H), 1.01 (t, *J* = 7.3 Hz, 3H). MS (ESI) m/z: 385 [M+H⁺].

### Preparation of examples 20 and 21 according to general route 3

### Example 20

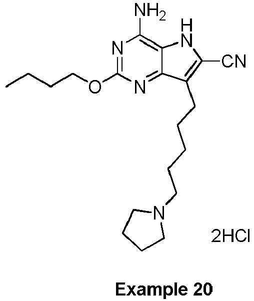

### 4-amino-2-butoxy-7-(5-(tetrahydropyrrol-1-yl)pentyl)-5H-pyrrolo[3,2-d]pyrimidine-6 -carbonitrile hydrochloride

### Route: Preparation of Example 20

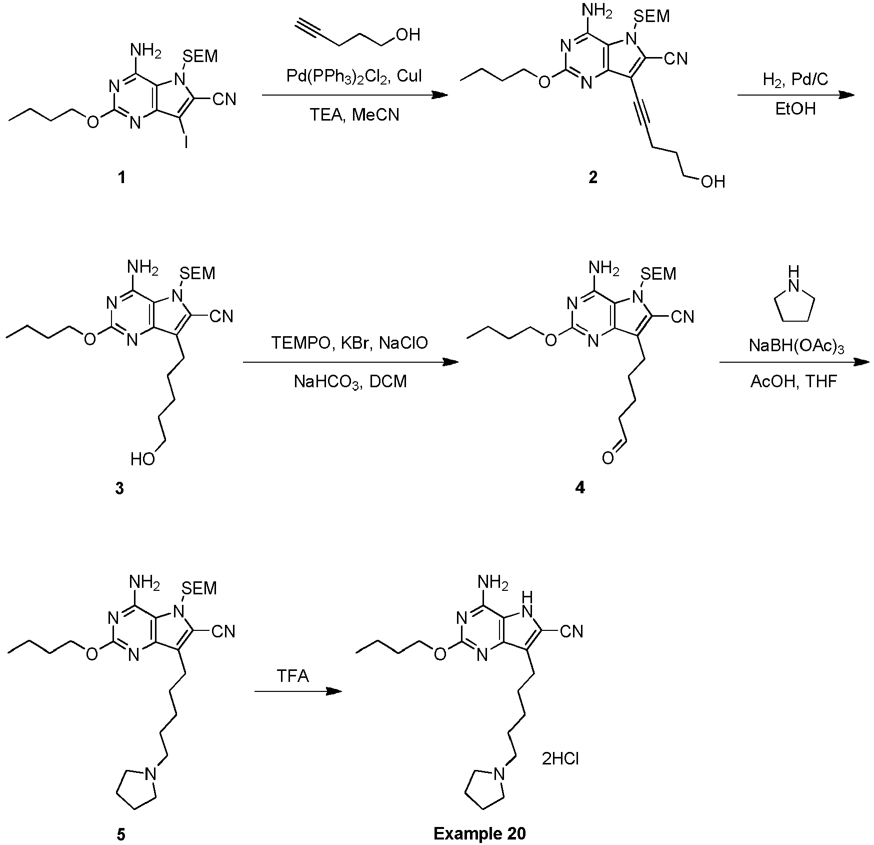

### Step A: synthesis of 4-amino-2-butoxy-7-(5-hydroxypent-1-yn-1-yl)-5-((2-(trimethylsilyl)ethoxy)methyl)-pyrrolo[3,2-d]pyrimidine-6-carbonitrile

A mixture of 4-amino-2-butoxy-7-iodo-5-((2-(trimethylsilyl)ethoxy)methyl)pyrrolo [3,2-*d*]pyrimidine-6-carbonitrile (1.5g, 3.08mmol), pent-4-yn-1-ol (0.52g, 6.15mmol), bis(triphenylphosphino)palladium (II) dichloride (43.2mg, 61.55µmol), copper(I) iodide (58.61mg, 307.75µmol) and triethylamine (0.93g, 9.23mmol) was placed in acetonitrile (25mL). The mixture was purged with nitrogen and stirred at 15 to 20 °C for 14 h. Then the mixture was poured into water (50mL) and extracted with ethyl acetate (50mL × 2). The combined organic phase was washed with saturated aqueous solution of ammonium chloride (50mL), dried over anhydrous sodium sulfate, filtered and the filtrate was concentrated under vacuum. The residue was purified by silica gel chromatography (column height: 250mm, diameter: 100mm, 100-200 mesh silica gel, Petroleum ether/Ethyl acetate=3/1, 1/1) to afford 4-amino-2-butoxy-7-(5-hydroxypent-1-yn-1-yl)-5-((2-(trimethylsilyl)ethoxy)methyl)-pyrrolo[3,2-*d*]pyrimidine-6-carbonitrile (1.20g, yield: 87.83%). MS (ESI) m/z: 444 [M+H⁺] .

### Step B: synthesis of 4-amino-2-butoxy-7-(5-hydroxypentyl)-5-((2-(trimethylsilyl) ethoxy)methyl)pyrrolo[3,2-d]pyrimidine-6-carbonitrile

4-Amino-2-butoxy-7-(5-hydroxypent-1-yn-1-yl)-5-((2-(trimethylsilyl)ethoxy)methyl) pyrrolo[3,2-*d*]pyrimidine-6-carbonitrile (1.20g, 2.71mmol) was dissolved in ethanol (40mL), Pd/C (50mg, 10%) was added in one portion under nitrogen atmosphere. The reaction mixture was purged with hydrogen gas for five times followed by stirring under 15 psi hydrogen atmosphere for 14h at 15 to 20°C. TLC indicated the reaction was completed. The reaction mixture was filtered with celite pad and the filter cake was washed with dichloromethane for 5 times. The combined filtrate was concentrated under vacuum to afford 4-amino-2-butoxy-7-(5-hydroxypentyl)-5-((2-(trimethylsilyl)ethoxy)methyl)pyrrolo[3 ,2-d]pyrimidine-6-carbonitrile (1.10, yield: 90.77%) as a yellow solid, which was used for next step directly. MS (ESI) m/z: 448 [M+H⁺].

### Step C: synthesis of 4-amino-2-butoxy-7-(5-hydroxypentyl)-5-((2-(trimethylsilyl)ethoxy)methyl)pyrrolo[3 ,2-d]pyrimidine-6-carbonitrile

4-Amino-2-butoxy-7-(5-hydroxypentyl)-5-((2-(trimethylsilyl)ethoxy)methyl)pyrrolo[ 3,2-*d*]pyrimidine-6-carbonitrile (0.85g, 1.90mmol) was dissolved in dichloromethane (50mL) at 0 to 5°C, saturated aqueous solution of sodium bicarbonate (34mL), TEMPO (597.19mg, 3.80mmol), potassium bromide (384.14mg, 3.23mmol) and aqueous solution of sodium hypochlorite (1.3M, 4.38mL) were added sequentially. The mixture was stirred at 0 to 5°C for 1h, and then poured into saturated aqueous solution of sodium thiosulfate (50mL) and extracted with dichloromethane (50mL × 2). The combined organic phase was washed with saturated aqueous solution of sodium thiosulfate (50mL) once, saturated aqueous solution of ammonium chloride (50mL × 3) and saturated aqueous solution of brine (75mL) once, the organic layer was dried over anhydrous sodium sulfate, filtered and the filtrate was concentrated under vacuum to afford 4-amino-2-butoxy-7-(5-oxopentyl)-5-((2-(trimethylsilyl) ethoxy)methyl)pyrrolo[3,2-*d*]pyrimidine-6-carbonitrile (600mg, crude) as a yellow solid which was used for the next step directly. MS (ESI) m/z: 446 [M+H⁺].

### Step D: synthesis of 4-amino-2-butoxy-7-(5-pyrrolidin-1-yl)pentyl)-5-((2-(trimethylsilyl)ethoxy)methyl)pyrrolo[3,2-d]pyrimidine-6-carbonitrile

4-Amino-2-butoxy-7-(5-oxopentyl)-5-((2-(trimethylsilyl)ethoxy)methyl)pyrrolo [3,2-*d* ]pyrimidine-6-carbonitrile (130mg, 291.72µmol) and pyrrolidine (41.49mg, 583.44µmol) were dissolved in tetrahydrofuran (5mL), sodium triacetoxyborohydride (154.57mg, 729.30µmol) and acetic acid (2-3 drops) were added sequentially. The mixture was stirred at 15 to 20 °C for 14 h, and then poured into saturated aqueous solution of sodium bicarbonate (30mL) and extracted with ethyl acetate (20mL × 2). The combined organic phase was washed with aqueous brine (30mL), dried over anhydrous sodium sulfate, filtered and the filtrate was concentrated under vacuum to afford 4-amino-2-butoxy-7-(5-(pyrrolidin-1-yl)pentyl)-5-((2-(trimethylsilyl)ethoxy) methyl)pyrrolo[3,2-*d*]pyrimidine-6-carbonitrile (146mg, crude), which was used for the next step directly. MS (ESI) m/z: 501 [M+H⁺].

### Step E: synthesis of 4-amino-2-butoxy-7-(5-(pyrrolidin-1-yl)pentyl)-5H-pyrrolo[3,2-d] pyrimidine-6-carbonitrile hydrochloride

4-Amino-2-butoxy-7-(5-(pyrrolidin-1-yl)pentyl)-5-((2-(trimethylsilyl)ethoxy)methyl)-pyrrolo[3,2-*d*]pyrimidine-6-carbonitrile (146.00mg, 291.56µmol) was dissolved in trifluoroacetic acid (2mL) and stirred at 15 to 20°C for 14 h. Then the mixture was poured into saturated aqueous solution of sodium bicarbonate (30mL) and extracted with ethyl acetate (20mL × 2). The combined organic phase was washed with saturated brine (30mL), dried over anhydrous sodium sulfate, filtered and the filtrate was concentrated in vacuum. The residue was purified by pre-HPLC (HCl condition) and lyophilized to afford 4-amino-2-butoxy-7-(5-(pyrrolidin-1-yl)pentyl)-5*H-*pyrrolo [3,2-*d*]pyrimidine-6-carbonitrile hydrochloride (53.03mg, yield: 49.09%) as a white solid. **¹H** NMR (400 MHz, METHANOL-*d*4): δ 4.57 (t, *J* = 6.5 Hz, 2H), 3.65 (br. s., 2H), 3.23-3.16 (m, 2H), 3.08 (d, *J* = 7.3 Hz, 2H), 2.82 (t, *J* = 7.4 Hz, 2H), 2.19-2.10 (m, 2H), 2.08-1.99 (m, 2H), 1.86-1.69 (m, 6H), 1.50 (dt, *J* = 7.7, 15.3 Hz, 4H), 1.01 (t, *J* = 7.4 Hz, 3H). MS (ESI) m/z: 371 [M+H⁺].

### Example 21

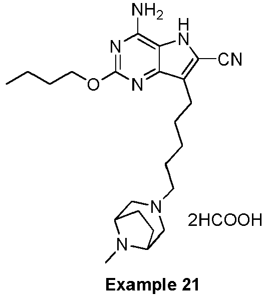

### 4-amino-2-butoxy-7-(5-(8-methyl-3,8-diazabicyclo[3.2.1]octan-3-yl)pentyl)-5H-pyrro lo[3,2-d]pyrimidine-6-carbonitrile formate

### Route: Preparation of Example 21

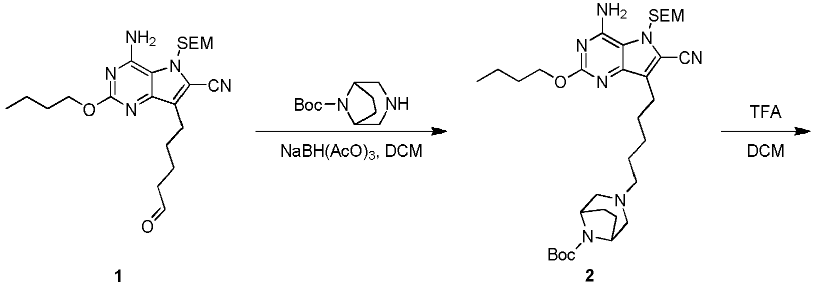

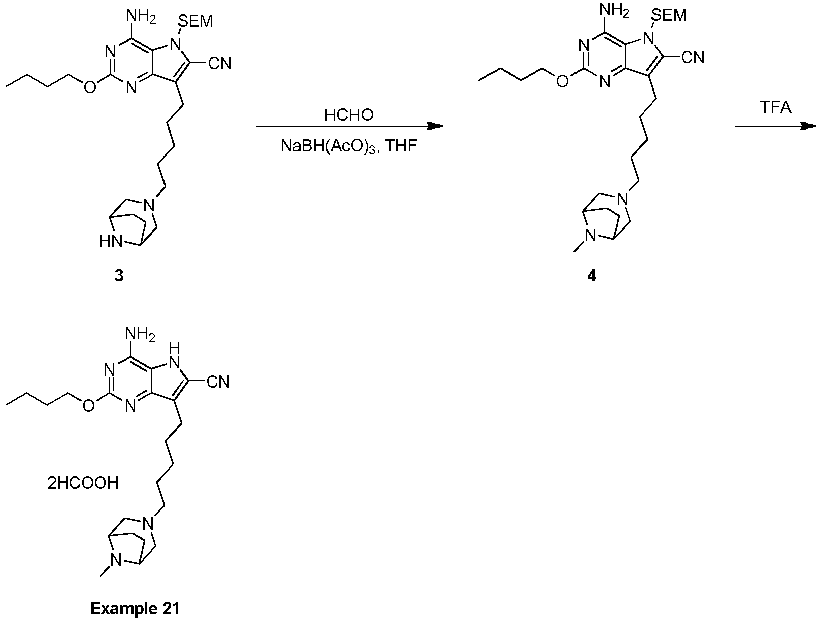

### Step A: synthesis of 4-amino-2-butoxy-7-(5-(8-tert-butoxycarbonyl-3,8-diazabicyclo[3.2.1]octan-3-yl)pentyl)-5-((2-(trimehtylsilyl)ethxoy)methyl)pyrrolo[3,2 -d]pyrimidine-6-carbonitrile formate

4-Amino-2-butoxy-7-(5-(8-*tert*-butoxycarbonyl-3,8-diazabicyclo[3.2.1]octan-3-yl)pen tyl)-5-((2-(trimehtylsilyl)ethxoy)methyl)pyrrolo[3,2-*d*]pyrimidine-6-carbonitrile was synthesized according to step D in example 20, except for replacing pyrrolidine with 8-*tert-*butoxycarbonyl-3,8-diazabicyclo[3.2.1]octane.

### Step B: synthesis of 4-amino-2-butoxy-7-(5-(3,8-diazabicyclo[3.2.1]octan-3-yl) pentyl)-5-((2-(trimehtylsilyl)ethxoy)methyl)pyrrolo[3,2-d]pyrimidine-6-carbonitrile

4-Amino-2-butoxy-7-(5-(8-tert-butxoycarbonyl-3,8-diazabicyclo[3.2.1]octan-3-yl)pen tyl)-5-((2-(trimehtylsilyl)ethxoy)methyl)pyrrolo[3,2-*d*]pyrimidine-6-carbonitrile (140.00mg, 218.1µmol) was dissolved in a mixture of dichloromethane (8mL) and trifluoroacetic acid (1mL). The reaction mixture was stirred at 10 to 20°C for 14 h, and then poured in saturated aqueous solution of sodium bicarbonate (30mL) slowly and extracted with dichloromethane (20mL × 2). The combined organic phase was washed with saturated brine (30mL), dried with anhydrous sodium sulfate, filtered and the filtrate was concentrated under vacuum to afford 4-amino-2-butoxy-7-(5-(3,8-diazabicyclo[3.2.1]octan-3-yl)pentyl)-5-((2-(trimehtylsil yl)ethxoy)methyl)pyrrolo[3,2-*d*]pyrimidine-6-carbonitrile (110mg, crude), which was used for the next step directly. MS (ESI) m/z: 542 [M+H⁺].

### Step C: synthesis of 4-amino-2-butoxy-7-(5-(3-methyl-3,8-diazabicyclo[3.2.1]octan-3-yl)pentyl)-5-((2-(trimehtylsilyl)ethxoy)methyl)pyrrolo[3,2-d]pyrimidine-6-carbonitr ile

4-Amino-2-butoxy-7-(5-(3,8-diazabicyclo[3.2.1]octan-3-yl)pentyl)-5-((2-(trimehtylsil yl)ethxoy)methyl)pyrrolo[3,2-*d*]pyrimidine-6-carbonitrile (110mg, 203.03µmol) was dissolved in tetrahydrofuran (5mL). Then sodium triacetoxyborohydride (154.57mg, 729.30µmol), 37% aqueous solution of formaldehyde (12.19mg, 406.06µmol) and catalytic amount of acetic acid were added sequentially. The reaction mixture was stirred at 15 to 20°C for 14 h, poured into saturated aqueous solution of sodium bicarbonate (30mL) and then extracted with ethyl acetate (25mL × 2). The combined organic phase was washed with saturated brine (30mL), dried over anhydrous sodium sulfate, filtered and the filtrate was concentrated under vacuum to afford 4-amino-2-butoxy-7-(5-(3-methyl-3,8-diazabicyclo[3.2.1]octan-3-yl)pentyl)-5-((2-(trimethylsilyl)ethoxy)methyl)pyrrolo[3,2-*d*]pyrimidine-6-carbonitrile (110mg, crude), which was used for the next step directly. MS (ESI) m/z: 556 [M+H⁺].

### Step D: synthesis of 4-amino-2-butoxy-7-(5-(3-methyl-3,8-diazabicyclo[3.2.1] octan-8-yl)pentyl)-5H-pyrrolo[3,2-d]pyrimidine-6-carbonitrile formate

4-Amino-2-butoxy-7-(5-(3-methyl-3,8-diazabicyclo[3.2.1]octan-8-yl)pentyl)-5-((2-(tr imethylsilyl)ethoxy)methyl)pyrrolo[3,2-d]pyrimidine-6-carbonitrile (110mg, 197.90µmol) was dissolved in trifluoroacetic acid (2mL). The reaction mixture was stirred at 15 to 20 °C for 14 h and then concentrated under vacuum. The residue was poured in saturated aqueous solution of sodium bicarbonate (30mL) and then extracted with a mixture of dichloromethane/methanol (10/1, v/v) (20mL × 3). The combined organic phase was dried over anhydrous sodium sulfate, filtered and the filtrate was concentrated under vacuum. The residue was purified by prep-HPLC (FA condition) and lyophilized to afford 4-amino-2-butoxy-7-(5-(3-methyl-3,8-diazabicyclo[3.2.1]octan-8-yl)pentyl)-5*H*-pyrro lo[3,2-*d*]pyrimidine-6-carbonitrile formate (24.80mg, yield: 26.57%) as a yellow solid. **¹H** NMR (400 MHz, METHANOL-d*4*): δ 8.33 (s, 2H), 4.34 (t, *J* = 6.6 Hz, 2H), 3.80 (br. s., 2H), 2.88- 2.78 (m, 4H), 2.76 (s, 3H), 2.48-2.38 (m, 4H), 2.14-1.93 (m, 4H), 1.79-1.70 (m, 4H), 1.55-1.46 (m, 4H), 1.42-1.34 (m, 2H), 0.99 (t, *J* = 7.4 Hz, 3H). MS (ESI) m/z: 426 [M+H⁺].

### Preparation of examples 22 to 25 according to general route 4

### Example 22

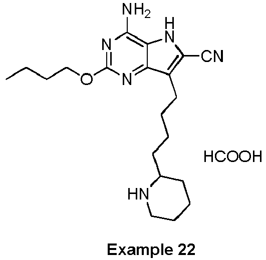

### 4-amino-2-butoxy-7-(4-(piperidin-2-yl)butyl)-5H-pyrrolo[3,2-d]pyrimidine-6-carboni trile formate

### Step A: synthesis of 4-amino-2-butoxy-7-(4-(2-(1-tert-butoxycarbonyl)-piperidyl) butyl)-5-((2-(trimethylsilyl)ethoxy)methyl)pyrrolo[3,2-d]pyrimidine-6-carbonitrile

4-Amino-2-butoxy-7-(4-(2-(1-*tert*-butoxycarbonyl)-piperidyl)butyl)-5-((2-(trimethylsi lyl)ethoxy)methyl)pyrrolo[3,2-*d*]pyrimidine-6-carbonitrile was synthesized according to steps E and F in example 1, except for replacing 4-amino-2-butoxy-7-iodo-5-((2-(trimethylsilyl)ethoxy)methyl)pyrrolo[3,2-*d*]pyrimidi ne with 4-amino-2-butoxy-7-iodo-5-((2-(trimethylsilyl)ethoxy)methyl) pyrrolo[3,2-*d*]pyrimidine-6-carbonitrile, and replacing 1-(pent-1'-ynyl)piperidine with 4-(2-(1-*tert*-butoxycarbonyl)piperidinyl)-1-butyne.

### Step B: synthesis of 4-amino-2-butoxy-7-(4-(piperidin-2-yl)butyl)-5-((2-(trimethylsilyl)ethoxy)methyl)pyrrolo[3,2-d]pyrimidine-6-carbonitrile

4-Amino-2-butoxy-7-(4-(2-(1-*tert*-butoxycarbonyl)-piperidyl)butyl)-5-((2-(trimethylsi lyl)ethoxy)methyl)pyrrolo[3,2-*d*]pyrimidine-6-carbonitrile (900.00mg, 1.50mmol) was dissolved in trifluoroacetic acid (2mL) and dichloromethane (20mL). The reaction mixture was stirred at room temperature for 12 h. LC-MS showed that the conversion was complete. The reaction mixture was neutralised with saturated aqueous solution of sodium bicarbonate (20mL) and then extracted with dichloromethane (30mL × 3). The combined organic phase was dried over anhydrous sodium sulfate, filtered and the filtrate was concentrated under vacuum to afford 4-amino-2-butoxy-7-(4-(piperidin-2-yl)butyl)-5-((2-(trimethylsilyl)ethoxy) methyl)pyrrolo[3,2-*d*]pyrimidine-6-carbonitrile (700.00mg, crude) as a white solid, which was used for the next step directly.

### Step C: synthesis of 4-amino-2-butoxy-7-(4-(piperidin-2-yl)butyl)-5H-pyrrolo [3,2-d]pyrimidine-6-carbonitrile formate

4-Amino-2-butoxy-7-(4-(piperidin-2-yl)butyl)-5*H*-pyrrolo[3,2-*d*]pyrimidine-6-carbon itrile formate was synthesized according to step J in example 4, except for replacing 4-amino-2-butoxy-7-(5-(1-morpholino)pentyl)-5-((2-(trimethylsilyl)ethoxy)methyl)py rrolo[3,2-*d*]pyrimidine-6-carbonitrile with 4-amino-2-butoxy-7-(4-(piperidin-2-yl) butyl)-5-((2-(trimethylsilyl)ethoxy)methyl)pyrrolo[3,2-*d*]pyrimidine-6-carbonitrile. **¹H** NMR (400 MHz, METHANOL-*d*₄): δ 8.42 (br. s., 1H), 4.34 (t, *J* = 6.53 Hz, 2H), 3.38 (br. s., 1H), 2.91-3.14 (m, 2H), 2.84 (t, *J* = 7.15 Hz, 2H), 1.34-2.10 (m, 16H), 1.01 (t, *J* = 7.40 Hz, 3H). MS (ESI) m/z: 371 [M+H⁺].

### Example: 4-(2-(1-tert-butoxycarbonyl)piperidyl)-1-butyne

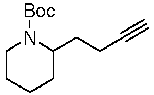

### 4-(2-(1-tert-butoxycarbonyl)piperidyl)-1-butyne

4-(2-(1-*Tert*-butoxycarbonyl)piperidyl)-1-butyraldehyde (1.70g, 7.04mmol) was dissolved in anhydrous methanol (30mL), dimethyl (1-diazo-2-oxopropyl) phosphonate (1.35g, 7.04mmol) was added at room temperature. The mixture was stirred at room temperature for 12 h. TLC showed the reaction was complete. The mixture was quenched by water (30mL) and extracted with dichloromethane (30mL × 3). The combined organic phase was dried over anhydrous sodium sulfate, filtered and the filtrate was concentrated under vacuum to afford 4-(2-(1-*tert*-butoxycarbonyl)piperidyl)-1-butyne. ¹**H** NMR (400 MHz, CHLOROFORM-*d*: δ 4.32 (d, *J* = 4.02 Hz, 1H), 4.00 (br. s., 1H), 3.69 (s, 1H), 2.77 (t, *J* = 12.67 Hz, 1H), 2.17 (td, *J* = 3.14, 7.53 Hz, 1H), 1.94-2.04 (m, 1H), 1.24-1.83 (m, 17H).

### Example 23

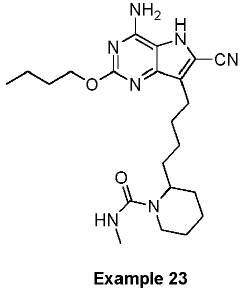

### 4-amino-2-butoxy-7-(4-(1-(1-N-methylcarbonyl)piperidin-2-yl)butyl)-5H-pyrrolo[3,2-d]pyrimidin-6-nitrile

### Step A: synthesis of 4-amino-2-butoxy-7-(4-(2-(1-N-methylcarbonyl)piperidinyl) butyl)-5-((2-(trimethylsilyl)ethoxy)methyl)pyrrolo[3,2-d]pyrimidin-6-nitrile

Under nitrogen atmosphere, 4-amino-2-butoxy-7-(4-(piperidin-2-yl)butyl)-5-((2-(trimethylsilyl)ethoxy)methyl)pyr rolo[3,2-*d*]pyrimidine-6-carbonitrile (200.00mg, 399.40µmol) and triethylamine (60.62mg, 599.10µmol) were dissolved in anhydrous dichloromethane (10mL), *N-*methylformyl chloride (44.82mg, 479.28µmol) was added dropwise. After the addition, the reaction mixture was stirred at room temperature for 1 h. LC-MS showed that the reaction was complete. The reaction mixture was quenched by 20mL saturated aqueous solution of sodium bicarbonate and extracted with 30mL dichloromethane for three times. The organic phase was dried over anhydrous sodium sulfate, filtered and the filtrate was evaporated under vacuum to afford 4-amino-2-butoxy-7-(4-(2-(1-*N*-methylcarbonyl)piperidinyl)butyl)-5-((2-(trimethylsil yl)ethoxy)methyl)pyrrolo[3,2-*d*]pyrimidin-6-nitrile (200mg, crude), which was used for the next step directly.

### Step B: synthesis of 4-amino-2-butoxy-7-(4-(1-(1-N-methylcarbonyl)piperidin-2-yl)butyl)-5H-pyrrolo[3,2-d]pyrimidin-6-nitrile

4-amino-2-butoxy-7-(4-(1-(1-*N*-methylcarbonyl)piperidin-2-yl)butyl)-5H-pyrrolo[3,2-*d*]pyrimidin-6-nitrile was synthesized according to step J in example 4, except for replacing 4-amino-2-butoxy-7-(5-(1-morpholino)pentyl)-5-((2-(trimethylsilyl)ethoxy) methyl)pyrrolo[3,2-*d*]pyrimidine-6-carbonitrile with 4-amino-2-butoxy-7-(4-(2-(1-N-methylcarbonyl)piperidyl)butyl)-5-((2-(trimethylsilyl)ethoxy)methyl)pyrrolo [3,2-*d*]py rimidin-6-nitrile. ¹**H** NMR (300 MHz, METHANOL-*d*₄: δ 8.20 (s, 1H), 4.39 (t, *J* = 6.53 Hz, 2H), 4.12 (br. s., 1H), 3.77 (d, *J* = 12.80 Hz, 1H), 2.70-2.95 (m, 6H), 1.46-1.88 (m, 12H), 1.19-1.44 (m, 3H), 1.01 (t, *J* = 7.40 Hz, 3H). MS (ESI) m/z: 428 [M+H⁺].

### Example 24

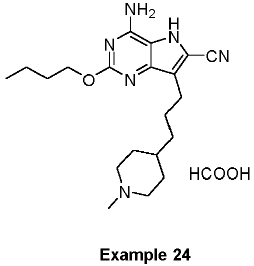

### 4-amino-2-butoxy-7-(3-(1-methylpiperidin-4-yl)propyl)-5H-pyrrolo[3,2-d]pyrimidine -6-carbonitrile formate

### Step A: synthesis of 4-amino-2-butoxy-7-(3-(1-tert-butoxycarbonylpiperidin-4-yl) propyl)-5-((2-(trimethylsilyl)ethoxy)methyl)pyrrolo[3,2-d]pyrimidine-6-carbonitrile

4-Amino-2-butoxy-7-(3-(1-*tert*-butoxycarbonylpiperidin-4-yl)propyl)-5-((2-(trimethyl silyl)ethoxy)methyl)pyrrolo[3,2-*d*]pyrimidine-6-carbonitrile was synthesized according to steps E and F in example 1, except for replacing 4-amino-2-butoxy-7-iodo-5-((2-(trimethylsilyl)ethoxy)methyl)pyrrolo[3,2-*d*]pyrimidi ne with 4-amino-2-butoxy-7-iodo-5-((2-(trimethylsilyl)ethoxy)methyl)pyrrolo [3,2-*d*]pyrimidine-6-nitrile, and replacing 1-(pent-1'-ynyl)piperidine with 3-(1-*tert*-butoxycarbonyl-4-piperidyl)propyne.

### Step B: synthesis of 4-amino-2-butoxy-7-(3-(1-methylpiperidin-4-yl)propyl)-5H-pyrrolo[3,2-d]pyrimidine-6-carbonitrile formate

4-Amino-2-butoxy-7-(3-(1-methylpiperidin-4-yl)propyl)-5*H-*pyrrolo[3,2-*d*]pyrimidin e-6-carbonitrile formate was synthesized according to steps B-D in example 21, except for replacing 4-amino-2-butoxy-7-(5-(8-*tert*-butoxycarbonyl-3,8-diazabicyclo[3.2.1]octan-3-yl)pent yl)-5-((2-(trimethylsilyl)ethoxy)methyl)pyrrolo[3,2-*d*]pyrimidin-6-nitrile with 4-amino-2-butoxy-7-(3-(1-*tert*-butoxycarbonyl-4-piperidin)propyl)-5-((2-(trimethylsil yl)ethoxy)methyl)pyrrolo[3,2-*d*]pyrimidin-6-nitrile. ¹**H** NMR (400 MHz, METHANOL-*d*₄): δ 8.51 (br. s., 1H), 4.31 (t, *J* = 6.5 Hz, 2H), 3.43 (d, *J* = 12.3 Hz, 2H), 2.93 (t, *J* = 12.2 Hz, 2H), 2.83-2.77 (m, 5H), 1.97 (d, *J* = 14.3 Hz, 2H), 1.81-1.34 (m, 11H), 0.99 (t, *J* = 7.4 Hz, 3H). MS m/z: 371 [M+H⁺].

### Example: synthesis of 3-(1-tert-butoxycarbonyl-4-piperidyl)propyne

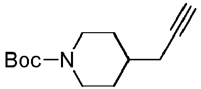

### 3-(1-tert-butoxycarbonyl-4-piperidyl)propyne

### Route: Preparation of 3-(1-tert-butoxycarbonyl-4-piperidyl)propyne

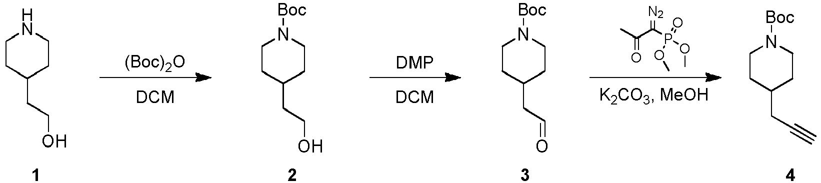

### Step A: synthesis of 2-(1-tert-butoxycarbonyl-4-piperidyl)ethanol

2-(Piperidin-4-yl)ethan-1-ol (3.00g, 23.22mmol) was dissolved in 30mL dichloromethane. Di-*tert*-butyl dicarbonate (5.22g, 23.92mmol) was slowly added portionwise at 15 to 20 °C. After that, the reaction mixture was stirred for 20 h at room temperature. The reaction mixture was slowly poured into 50mL water after the reaction was complete as monitored by TLC. Then, the mixture was extracted with dichloromethane (50mL × 2). The combined organic layer was dried over anhydrous sodium sulfate, filtered and the filtrate was concentrated under vacuum to give a residue. The residue was purified by column chromatography (Height: 250mm, Diameter, 20mm, 100-200 slica gel, petroleum/ethyl acetate = 3:1, 1:1) to afford 2-(1-*tert*-butoxycarbonyl-4-piperidyl)ethanol (4.89g, 91.83% yield) as a colorless liquid.

### Step B: synthesis of 2-(1-tert-butoxycarbonyl-4-piperidyl)acetaldehyde

2-(1-*Tert*-butoxycarbonyl-4-piperidyl)ethanol (4.89g, 21.32mmol) was dissolved in 50mL dichloromethane, Dess-Martin oxidant (9.95g, 23.46mmol) was added in one portion. The reaction mixture was stirred for 2 h at room temperature. The reaction mixture was poured into a mixture of 250mL 10% sodium thiosulfate aquesou solution and 150mL saturated sodium bicarbonate aqueous solution and the mixture was stirred for 45 min. After that, the reaction mixture was extracted with dichloromethane (100mL × 3). The combined organic layer was washed with 150mL saturated sodium bicarbonate aqueous solution and 150mL saturated sodium chloride aqueous solution sequentially, dried over anhydrous sodium sulfate, filtered and the filtrate was concnetrated under vacuum to afford 2-(1-*tert*-butoxycarbonyl-4-piperidyl)acetaldehyde (4.85g, crude), which was used in next step directly.

### Step C: synthesis of 3-(1-tert-butoxycarbonyl-4-piperidyl)propyne

2-(1-*Tert*-butoxycarbonyl-4-piperidyl)acetaldehyde (4.85g, 21.34mmol) was dissolved in 50mL methanol, potassium carbonate (5.90g, 42.68mmol) was added. After the mixture was stirred at room temperatrue for 5 minutes, dimethyl (1-diazo-2-oxopropyl) phosphonate (4.10g, 21.34mmol) was added dropwise. After the addition, the reaction mixture was stirred for 17 h at room temperature, and then poured into the mixture of dichloromethane (250mL) and saturated aqueous solution of sodium bicarbonate (50mL). The organic layer was separated and filtered through celite pad. The filtrate was concentrated under vacuum. The residue was dissolved in ethyl acetate (50mL) and filtered through silica gel pad. The filter cake was washed with ethyl acetate (30mL × 3). The combined filtrate was concentrated under vacuum to afford 3-(1-*tert*-butoxycarbonyl-4-piperidyl)propyne (3.92g). ¹**H** NMR (400MHz, CHLOROFORM-*d*) δ 4.12 (d, *J* = 3.5 Hz, 2H), 2.69 (t, *J* = 11.8 Hz, 2H), 2.15 (dd, *J* = 2.6, 6.7 Hz, 2H), 1.99 (t, *J* = 2.6 Hz, 1H), 1.76 (d, *J* = 13.3 Hz, 3H), 1.46 (s, 9H), 1.20 (dq, *J* = 4.4, 12.3 Hz, 2H).

### Example 25

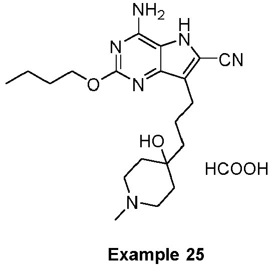

### 4-Amino-2-butoxy-7-(3-(1-methylpiperidin-4-yl)-3'-hydroxy-propyl)-5H-pyrrolo[3,2-d]pyrimidine-6-carbonitrile formate

### Route: Preparation of Example 25

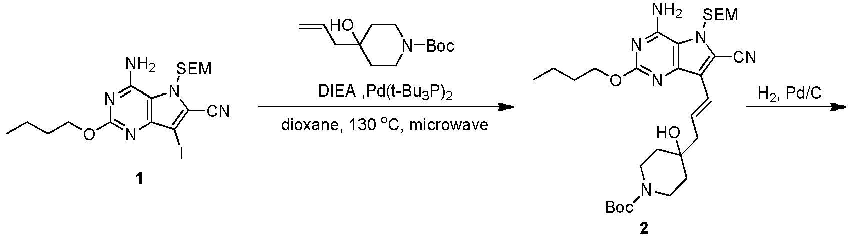

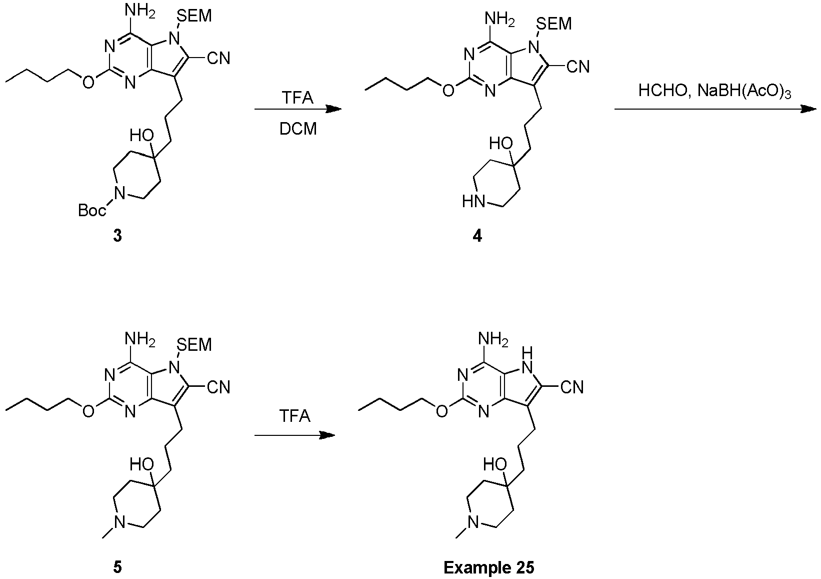

### Step A: synthesis of 4-amino-2-butoxy-7-(3-hydroxy-3-(1-tert-butylcarbonyl-4-piperidyl)allyl)-5-((2-(trimethylsilyl)ethoxy)methyl)pyrrolo[3,2-d]pyrimidin-6-nitrile

3-(1-*tert*-butoxycarbonyl-4-hydroxy-4-piperidyl)-1-proplene (400.00mg, 1.66mmol) and 4-amino-2-butoxy-7-iodo-5-((2-(trimethylsilyl)ethoxy)methyl)pyrrolo[3,2-*d*] pyrimidine-6-carbonitrile (734.79mg, 1.51mmol) were dissolved in anhydrous 1,4-dioxane (15mL), *N,N-*diisopropylethyl amine (386.17mg, 2.99mmol) and bis-(tri-*tert*-butylphosphine)palladium (16.97mg, 33.20µmol) were added in one portion under nitrogen atmosphere. The reaction was purged with nitrogen for 3 times and stirred at 130°C under microwave for 30 min. After LC-MS showed the reaction was complete, the reaction mixture was cooled to room temperature and poured into saturated aqueous solution of sodium bicarbonate (50mL). Then, the mixture was extracted with 60mL ethyl acetate. The organic layer was washed with 80mL saturated brine, dried over anhydrous sodium sulfate, filtered and the filtrate was concentrated under vacuum to give a residue. The residue was purified by pre-HPLC (trifluoroacetic acid condition) and lyophilized to give 4-amino-2-butoxy-7-(3-hydroxy-3-(1-*tert*-butylcarbonyl-4-piperidyl)allyl)-5-((2-(trim ethylsilyl)ethoxy)methyl)pyrrolo[3,2-*d*]pyrimidin-6-nitrile (160.00mg, 266.30µmol, yield: 16.04%) as a yellow solid.

### Step B: synthesis of 4-amino-2-butoxy-7-(3-hydroxy-3-(1-tert-butylcarbonyl-4 piperidyl)propyl)-5-((2-(trimethylsilyl)ethoxy)methyl)pyrrolo[3,2-d]pyrimidin-6-nitril e

4-Amino-2-butoxy-7-(3-hydroxy-3-(1-*tert*-butylcarbonyl-4-piperidyl)allyl)-5-((2-(tri methylsilyl)ethoxy)methyl)pyrrolo[3,2-*d*]pyrimidin-6-nitrile (230.0mg, 382.1µmol) was dissolved in tetrahydrofuran (25mL), the mixture was purged with nitrogen for 3 times and then 10% dry palladium charcoal (100mg) was added in one portion. The reaction mixture was purged with hydrogen for 3 times and stirred under hydrogen atmosphere (15psi) at 10-15°C for 14 h. After LC-MS showed the reaction was complete, the reaction mixture was filtered through celite pad. The filtrate was concentrated to remove the solvent to afford 4-amino-2-butoxy-7-(3-hydroxy-3-(1-*tert*-butylcarbonyl-4-piperidyl)propyl)-5-((2-(trimethylsilyl)ethoxy)methyl)pyrrolo [3,2-*d*]pyrimidin-6-nitrile (190.00mg, crude) as a yellow solid, which was used for the next step directly.

### Step C: synthesis of 4-amino-2-butoxy-7-(3-hydroxy-(4-piperidyl)propyl)-5-((2-(trimethylsilyl)ethoxy)methyl)pyrrolo[3,2-d]pyrimidine-6-carbonitrile

4-Amino-2-butoxy-7-(3-hydroxy-3-(1-*tert*-butoxycarbonyl-4-piperidyl)propyl)-5-((2-( trimethylsilyl)ethoxy)methyl)pyrrolo[3,2-*d*]pyrimidine-6-carbonitrile (100.00mg, 165.88µmol) was dissoved in trifluoroacetic acid (0.5mL) and dichloromethane (4mL). The reaction was stirred at 10 to 15°C for 4.5 h. After LC-MS showed the reaction was complete, the reaction was quenched with 20mL saturated sodium bicarbonate aqueous solution and extracted with 15mL dichloromethane. The organic layer was concentrated under vacuum to afford 4-amino-2-butoxy-7-(3-hydroxy-(4-piperidyl)propyl)-5-((2-(trimethylsilyl)ethoxy)met hyl)pyrrolo[3,2-*d*]pyrimidine-6-carbonitrile (80.00mg, crude) as a yellow solid, which was used for the next step directly.

### Step D: synthesis of 4-amino-2-butoxy-7-(3-hydroxy(1-methylpiperidin-4-yl) propyl)-5-((2-(trimethylsilyl)ethoxy)methyl)pyrrolo[3,2-d]pyrimidine-6-carbonitrile

4-Amino-2-butoxy-7-(3-hydroxy-(4-piperidyl)propyl)-5-((2-(trimethylsilyl)ethoxy)me thyl)pyrrolo[3,2-*d*]pyrimidine-6-carbonitrile (80.00mg, 159.13µmol) was dissoved in tetrahydrofuran (5mL), formaldehyde (14.34mg, 477.39µmol), acetic acid (9.56mg, 159.13µmol) and sodium borohydride acetate (84.32mg, 397.83µmol) were added sequentially. The reaction mixture was stirred under 10 to 15°C for 2 h. After LC-MS showed the reaction was complete, the reaction mixture was quenched by 15mL water and extracted with 20mL dichloromethane. The organic phase was dried over anhydrous sodium sulfate, filtered and the filtrate was concentrated under vacuum to afford 4-amino-2-butoxy-7-(3-hydroxy-(1-methylpiperidin-4-yl) propyl)-5-((2-(trimethylsilyl)ethoxy)methyl)pyrrolo [3,2-*d*]pyrimidine-6-carbonitrile (90.00mg, crude) as a yellow solid, which was used for the next step directly.

### Step E: synthesis of 4-amino-2-butoxy-7-(3-hydroxy-(1-methylpiperidin-4-yl)propyl)-5H-pyrrolo[3,2-d]pyrimidine-6-carbonitrile formate

4-Amino-2-butoxy-7-(3-hydroxy(1-methylpiperidin-4-yl)propyl)-5-((2-(trimethylsilyl )ethoxy)methyl)pyrrolo[3,2-*d*]pyrimidine-6-carbonitrile (90.00mg, 174.17µmol) was dissolved in trifluoroacetic acid (1.5mL). The mixture was stirred under 10 to 15°C for 5h. After LC-MS showed the reaction was complete, the mixture was concentrated to remove trifluoroacetic acid under vacuum. The residue was purified by pre-HPLC (FA condition) and lyophilized to afford 4-amino-2-butoxy-7-(3-hydroxy-(1-methylpiperidin-4-yl)propyl)-5*H-*pyrrolo[3,2-*d*]p yrimidine-6-carbonitrile formate (28.27mg). ¹**H** NMR (400 MHz, METHANOL-*d*₄: δ 8.24 (br. s., 1H), 4.36 (t, *J* = 6.5 Hz, 2H), 3.33-3.22 (m, 4H), 2.91-2.80 (m, 5H), 1.90-1.72 (m, 8H), 1.64-1.47 (m, 4H), 1.01 (t, *J* = 8.0 Hz, 3H). MS (ESI) m/z: 387 [M+H⁺] .

### Example: synthesis of 3-hydroxy-3-(1-tert-butoxycarbonyl-4-piperidyl)-1-propene

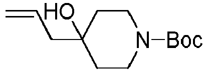

### 3-hydroxy-3-(1-tert-butoxycarbonyl-4-piperidyl)-1-propene

1-*Tert*-butoxycarbonyl-4-piperidone (2.5g, 12.55mmol) was dissolved in anhydrous tetrahydrofuran (25mL), allyl magnesium bromide (1M, 16.30mL) was slowly added dropwise under nitrogen atmosphere at 0°C over 1h. The reaction was stirred for 2 h at 0°C until the reaction was complete as monitored by TLC. The reaction mixture was quenched with saturated aqueous solution of ammonium chloride (80mL), the reaction was allowed to warm to room temperature and extracted with ethyl acetate (100mL). The organic phase was washed with saturated aqueous solution of sodium chloride (100mL) once and dried over anhydrous sodium sulfate, filtered, and the filtrate was concentrated under vacuum. The residue was purified with column chromatography (height: 100mm, diameter: 50mm, 100-200 silica gel, petroleum ether/ethyl acetate = 4/1, 2/1) to afford 3-hydroxy-3-(1-*tert*-butoxycarbonyl-4-piperidyl)-1-propene (750.00mg, 3.11mmol, yield: 19.08%) as a yellow liquid. ¹**H** NMR (400 MHz, CHLOROFORM-*d*): δ 5.93-5.83 (m, 1H), 5.27-5.14 (m, 2H), 3.83 (br. s., 2H), 3.18 (br. s., 2H), 2.25 (d, *J* = 7.5 Hz, 2H), 1.61-1.50 (m, 4H), 1.48 (s, 9H).

### Bioactivity Assay

### Test 1: TLR7 Agonist Screening Protocol in vitro

Reagents:
HEK-Blue-hTLR7 cells
DMEM media
Heat-Inactivated Fetal Bovine Serum
Anti-mycoplasma reagent Normocin
Zeocin
Blasticidin

### Protocol:

**1.** Preparation for 96-well plate: Compounds are 3 folds serial diluted with DMSO by POD, start from 10mM, 10 concentrations (column 2 to 11, duplicated), 1 µ L DMSO per well. Take 1 µ L of 5 mM R848 as positive control (column 12) and 1µL of DMSO as negative control (column 1).
**2.** Harvest cells and adjust cell suspension to the density of 250,000 cells/ml.
**3.** 200µL cells are seeded into the cell culture plate containing compounds with the cell density of 50,000 cells/well, the final DMSO concentration is 0.5%.
**4.** Incubate the plates containing the cells and compounds at 37°C, 5% CO₂ for 24 hours.
**5.** After 24 hours' incubation, fetch 20µL supernatant from each well to transfer to another transparent 96-well cell culture plate. Add 180µL QUANTI-Blue to each well of the cell culture plate containing 20µL supernatant, and incubate at 37°C, 5% CO₂ for 1 hour.
**6.** After 1 hour, determine alkaline phosphatase levels in 20µL supernatant by using ELIASA reading at OD650.
**7.** Data analysis by Prism software, EC50 of each compound was obtained.

| | 1 | 2 | 3 | 4 | 5 | 6 | 7 | 8 | 9 | 10 | 11 | 12 |
|---|---|---|---|---|---|---|---|---|---|---|---|---|
| A | | exa mple 1 | exa mple 1 | exa mple 1 | exa mple 1 | exa mple 1 | exa mple 1 | exa mple 1 | exa mple 1 | exa mple 1 | exa mple 1 | |
| B | | 5000 0.0 | 1666 6.7 | 5555 .6 | 1851 .9 | 617. 3 | 205. 8 | 68.6 | 22.9 | 7.6 | 2.5 | |
| C | | exa mple 2 | exa mple 2 | exa mple 2 | exa mple 2 | exa mple 2 | exa mple 2 | exa mple 2 | exa mple 2 | exa mple 2 | exa mple 2 | |
| D | 0.5 % | 5000 0.0 | 1666 6.7 | 5555 .6 | 1851 .9 | 617. 3 | 205. 8 | 68.6 | 22.9 | 7.6 | 2.5 | 25 µg /m |
| E | D MS O | exa mple 3 | exa mple 3 | exa mple 3 | exa mple 3 | exa mple 3 | exa mple 3 | exa mple 3 | exa mple 3 | exa mple 3 | exa mple 3 | 1 R8 48 |
| F | | 5000 0.0 | 1666 6.7 | 5555 .6 | 1851 .9 | 617. 3 | 205. 8 | 68.6 | 22.9 | 7.6 | 2.5 | |
| G | | exa mple 4 | exa mple 4 | exa mple 4 | exa mple 4 | exa mple 4 | exa mple 4 | exa mple 4 | exa mple 4 | exa mple 4 | exa mple 4 | |
| H | | 5000 | 1666 | 5555 | 1851 | 617. | 205. | 68.6 | 22.9 | 7.6 | 2.5 | |
| | | 0.0 | 6.7 | .6 | .9 | 3 | 8 | | | | | |

| | | | | | | | | | | | | |
|---|---|---|---|---|---|---|---|---|---|---|---|---|
| **Note: unit (nm/L)** | | | | | | | | | | | | |

The test results were shown as table 1.

**Table 1**

| **Example** | **EC50 (nM)** | **Example** | **EC50 (nM)** | **Example** | **EC50 (nM)** |
|---|---|---|---|---|---|
| **GSK-2245035** | A | **9** | B | **18** | A |
| **1** | A | **10** | D | **19** | A |
| **2** | A | **11** | B | **20** | A |
| **3** | A | **12** | A | **21** | B |
| **4** | A | **13** | A | **22** | B |
| **5** | A | **14** | A | **23** | B |
| **6** | A | **15** | B | **24** | A |
| 7 | C | **16** | B | **25** | D |
| **8** | A | **17** | A | | |

| | | | | | |
|---|---|---|---|---|---|
| Note: EC50 ranges were shown as follows: 100nM≥A≥1nM; 250nM≥B>100nM; 500nM≥C>250nM; 500nM >D. | | | | | |

As shown by the data, the examples of the present invention exhibited comparable TLR7 agonism activity to GSK-2245035.

### Test 2: PBMC Assay Protocol

Assays were conducted to determine cytokine stimulation at 24 hrs from human Peripheral Blood Mononuclear Cell (PBMC) using the compounds of the present invention. Cell supernatants were assayed directly for IFN-α and TNF-α without dilution. The compounds of the present invention were diluted 10 folds with the culture media from 20 mM DMSO solution to a total of 11 points. The compounds of the present invention were added in 50 µ L cell media in a 96-well plate at 9 points with 200µM as the highest concentration and fresh PBMCs were seeded with 450,000 cells in 150µL media per well. The plates were incubated at 37°C and 5% CO₂ for 24 hrs and then spun at 1200 rpm for 5 min, which was followed by collecting supernatant and storing at -20 °C . Cytokine secretion was assayed with BD Cytometric Bead Array (CBA) Flex Sets, using a Flow Cytometry. The IFN-α and TNF-α MEC value for a compound was the lowest concentration at which the compound stimulated cytokine level at least 3 folds over the lowest detection limit using the assay method above.

### Test Result:

### Example 1 and example 2, IFN-α: ≤0.001 nM; TNF-α: ≥1 nM.

### Test 3: Assay for the induction of Interferon-α (IFN-α) and Tumor Necrosis Factor-α (TNF-α) following intranasal dosing in the mouse

Female Balb/c mice (18-20g) were anesthetized with isoflurane and then administered intranasally (20 µ L in total between the nostrils) with compounds dissolved in saline with 0.2% Tween 80. After two hours, mice were euthanased by CO₂ inhalation and blood samples were taken by cardiac puncher. Then blood samples were centrifuged and serum was collected. Serum samples were tested by ELISA with appropriate dilution according to the manufacturer's instructions. In this model, compounds could induce different IFN-α/TNF-α levels related to the dose of the compounds, while no IFN-α/TNF-α was detected in vehicle treated controls.

| **Group** | **Mice per group** | **Compound treatment** | | | | | |
|---|---|---|---|---|---|---|---|
| | | **Compound** | **Conc.** (**mM**) | **Vol. (ul/mice)** | **Dose (mpk)** | **Schedule** | **Bleeding time** |
| 1 | 8 | Vehicle* | / | 5 | / | i.n, once | 2 h after dose, all the mice were bled for IFN-alpha/ TNF-alpha test |
| 2 | | GSK-2245035 | 1 | | 0.1 | | |
| 3 | | GSK-2245035 | 3 | | 0.3 | | |
| 4 | | GSK-2245035 | 10 | | 1 | | |
| 5 | | GSK-2245035 | 30 | | 3 | | |
| 6 | | Example 1 | 1 | | 0.1 | | |
| 7 | | Example 1 | 3 | | 0.3 | | |
| 8 | | Example 1 | 10 | | 1 | | |
| 9 | | Example 1 | 30 | | 3 | | |

The results were shown as Table 2.

**Table 2**

| | **Example** | **Conc. (1 mM) pg/mL** | **Conc. (3 mM) pg/mL** | **Conc. (10 mM) pg/mL** | **Conc. (30 mM) pg/mL** |
|---|---|---|---|---|---|
| **IFN-α** | **GSK-2245035** | <100 | 1000 | 3500 | 4500 |
| | **Example 1** | 500 | 4500 | 7500 | 7000 |
| **TNF-α** | **GSK-2245035** | / | <10 | 50 | 400 |
| | **Example 1** | / | 100 | 300 | 500 |

As shown from the data, compounds of the present invention induced higher concentration of interferon IFN-alpha than GSK2245035, and this advantage became more obvious under low dosing concentration.

## Claims

1. A compound of formula (I) or a pharmaceutically acceptable salt, a hydrate or a prodrug thereof, 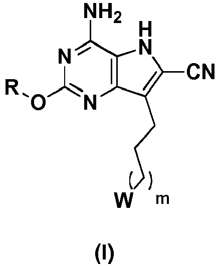 wherein,
R is an optionally substituted C₃₋₈ alkyl;
m is 0, 1, 2, 3, or 4;
W is an optionally substituted C₃₋₈ heteroalkyl, optionally substituted 4-12 membered cycloalkyl, optionally substituted 4-12 membered heterocycloalkyl, or optionally substituted amino acid;
the "hetero" represents heteroatom or heteroatomic group, which is independently selected from the group consisting of O, S, N, C(=O), S(=O) and S(=O)₂;
the number of heteroatom or heteroatomic group is independently 0, 1, 2, 3 or 4.

2. The compound of claim 1, wherein the substituent of C₃₋₈ alkyl, C₃₋₈ heteroalkyl, 4-12 membered cycloalkyl, or 4-12 membered heterocycloalkyl is independently selected from the group consisting of F, Cl, Br, I, OH, CN, NH₂, NH₂(C=O), C₁₋₄ alkyl or C₁₋₄ heteroalkyl, 4-6 membered alkyl or heteroalkyl, 4-6 membered heteroalkyl-C(=O)-, the 4-6 membered alkyl or heteroalkyl is optionally substituted by halogen, NH₂, OH, CN or C₁₋₄ alkyl; specifically, the substituent of C₃₋₈ alkyl, C₃₋₈ heteroalkyl, 4-12 membered cycloalkyl or 4-12 membered heterocycloalkyl is independently selected from the group consisting of F, Cl, Br, I, OH, CN, NH₂, NH₂(C=O), Me, Et, 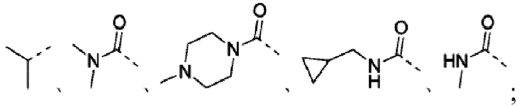 the "hetero" is the same as defined in claim 1;
the number of the substituent is independently 0, 1, 2 or 3.

3. The compound of claim 1 or 2, wherein the C₃₋₈ alkyl is selected from the group consisting of

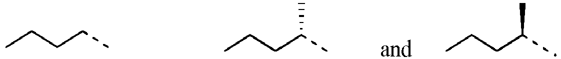

4. The compound of claim 1 or 2, wherein the C₃₋₈ heteroalkyl is -N(C₁₋₄ alkyl)(C₁₋₄ alkyl).

5. The compound of claim 1 or 2, wherein the 4-12 membered cycloalkyl or 4-12 membered heterocycloalkyl is 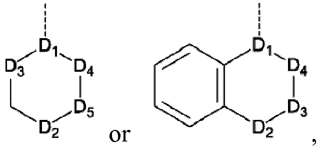 D₁ is N or C(Rₐ), D₂₋₅ is independently selected from the group consisting of O, S, [C(Rₐ)(R_{b})]₁₋₂ and N(R_{c}), one or both of D₃ and D₄ may be single bond(s), Ra, Rb, or Rc is independently selected from the group consisting of H, C₁₋₄ alkyl, C₁₋₄ heteroalkyl, halogen, OH, CN and NH₂(C=O), any Rₐ and R_{b} may be optionally attached to the same atom to form a 4-6 membered cycloalkyl, 4-6 membered oxa-cycloalkyl or 4-6 membered aza-cycloalkyl, the 4-6 membered cycloalkyl is optionally substituted by 1 to 3 of C₁₋₄ alkyl.

6. The compound of claim 1, wherein W is selected from the group consisting of

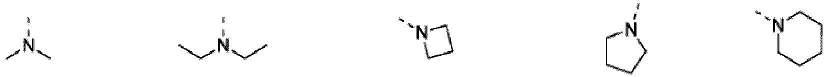

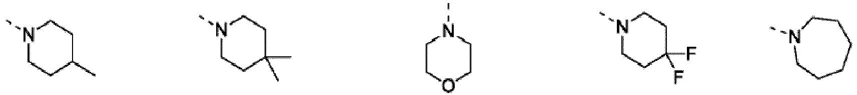

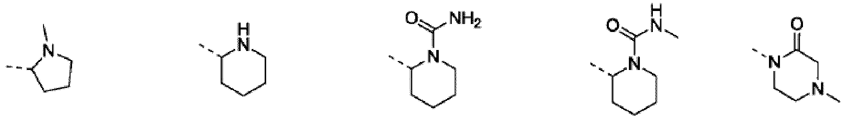

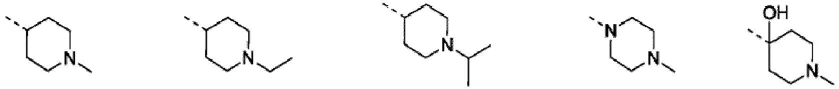

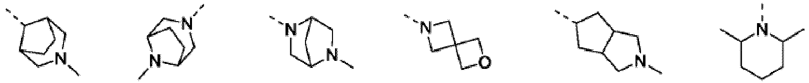

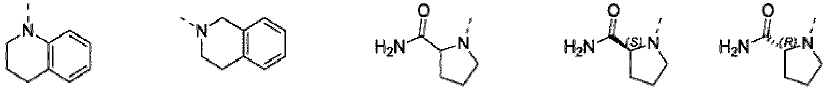

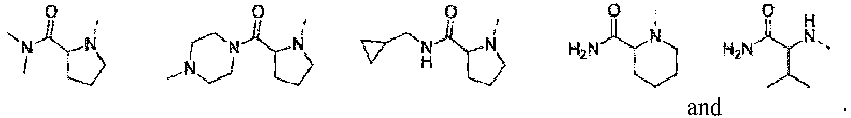

7. The compound of claim 1, wherein the compound is selected from the group consisting of

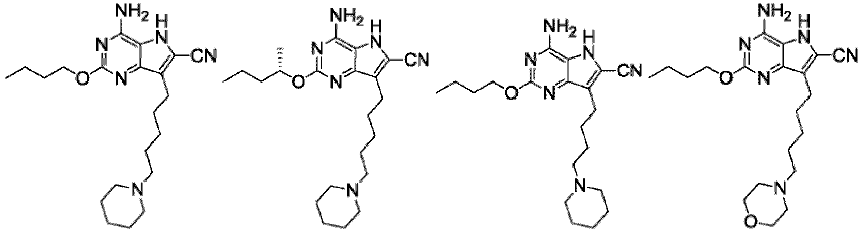

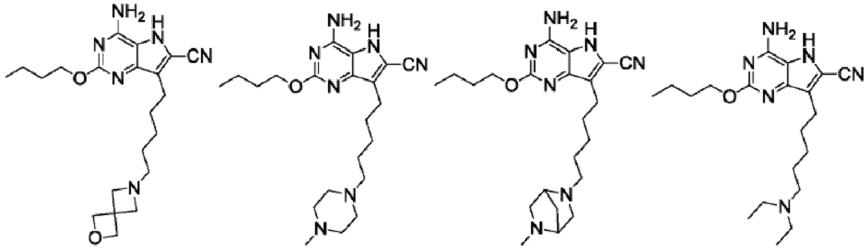

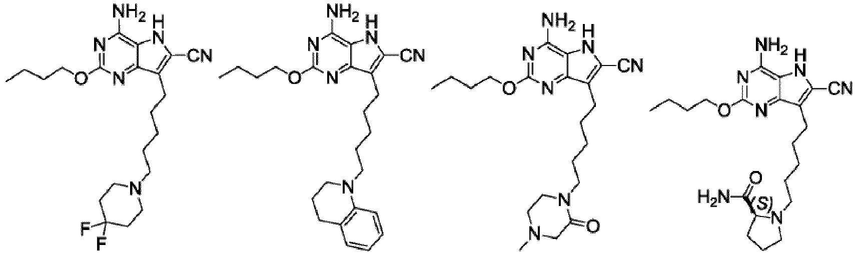

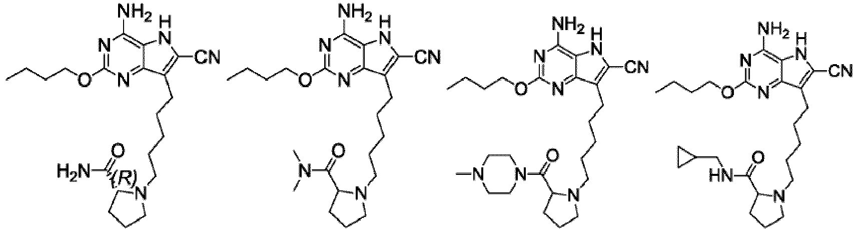

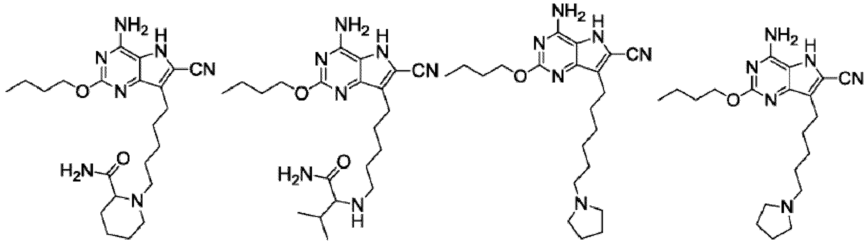

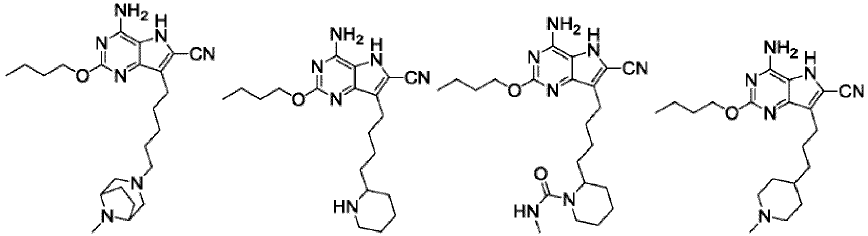

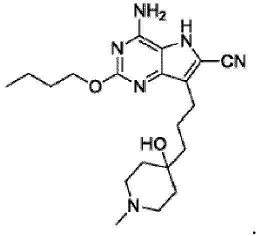

8. The compound of claim 1, whose process for preparing is as follows:
process 1: 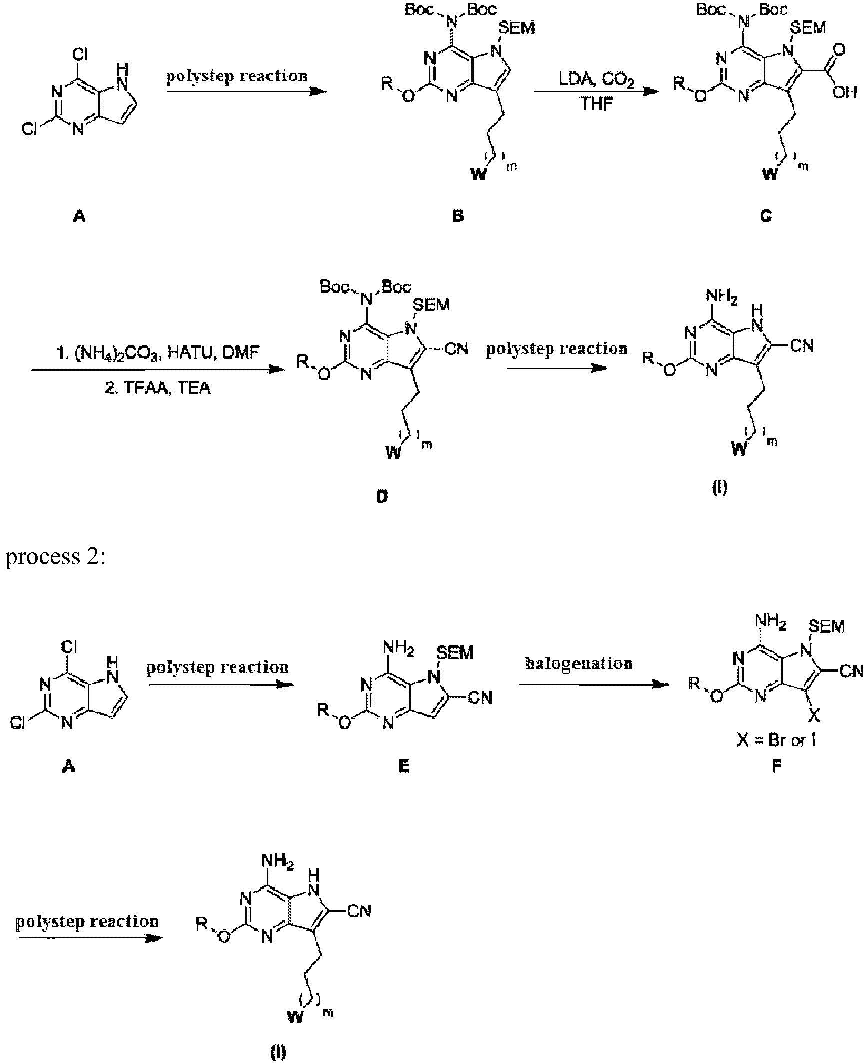
wherein, SEM represents 2-(trimethylsilyl)ethoxymethyl, which is an amino protecting group.

9. A use of the compound, pharmaceutically acceptable salt, hydrate, or prodrug thereof according to any one of claims 1 to 7 in manufacturing a medicament for the prevention or treatment of allergic diseases and other inflammatory conditions, infection diseases or cancers.

10. The use of claim 9, wherein the disease is allergic rhinitis or asthma.
